(19) Europäisches Patentamt
European Patent Office
Office européen des brevets

(11) Veröffentlichungsnummer : **0 369 219 B1**

(12) # EUROPÄISCHE PATENTSCHRIFT

(45) Veröffentlichungstag der Patentschrift : **04.01.95 Patentblatt 95/01**

(51) Int. Cl.[6] : **G03F 7/022,** C07C 309/26

(21) Anmeldenummer : **89119883.0**

(22) Anmeldetag : **26.10.89**

(54) **Neue, strahlungsempfindliche Verbindungen, hiermit hergestelltes strahlungsempfindliches Gemisch und Aufzeichnungsmaterial.**

(30) Priorität : **04.11.88 DE 3837500**

(43) Veröffentlichungstag der Anmeldung : **23.05.90 Patentblatt 90/21**

(45) Bekanntmachung des Hinweises auf die Patenterteilung : **04.01.95 Patentblatt 95/01**

(84) Benannte Vertragsstaaten : **BE CH DE FR GB LI**

(56) Entgegenhaltungen :
**EP-A- 0 178 356**
**EP-A- 0 283 898**
**EP-A- 0 287 750**
**CH-A- 341 071**
**DD-A- 263 982**
**DE-C- 171 024**

(73) Patentinhaber : **HOECHST AKTIENGESELLSCHAFT**
**D-65926 Frankfurt (DE)**

(72) Erfinder : **Scheler, Siegfried, Dr. Dipl.-Chem.**
**Stormstrasse 5**
**D-6200 Wiesbaden (DE)**
Erfinder : **Buhr, Gerhard, Dr. Dipl.-Chem.**
**Am Erdbeerstein 28**
**D-6240 Königstein (DE)**
Erfinder : **Lenz, Helmut, Dipl.-Ing.**
**Hochheimerstrasse 19**
**D-6234 Hattersheim 2 (DE)**
Erfinder : **Bergmann, Klaus**
**Buchenweg 57**
**D-65 Mainz-Bretzenheim (DE)**
Erfinder : **Siegel, Herbert, Dr. Dipl.-Chem.**
**Am Alten Birnbaum 10 a**
**D-6238 Hofheim/Ts. (DE)**

**EP 0 369 219 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue strahlungsempfindliche Verbindungen, hiermit hergestelltes Gemisch, insbesondere ein Photoresistgemisch. Das Gemisch enthält im wesentlichen ein wasserunlösliches, in wäßrig-alkalischen oder organischen Lösemitteln lösliches, zumindest aber quellbares harzartiges Bindemittel, gegebenenfalls einen durch Säure aktivierbaren Vernetzer und als strahlungsempfindliche Verbindung einen Ester oder ein Amid einer substituierten 1,2-Naphthochinon-(2)-diazid-4-sulfonsäure. Die Erfindung betrifft ferner ein Aufzeichnungsmaterial, das aus einem Schichtträger und aus dem strahlungsempfindlichen Gemisch hergestellt wird. Das Aufzeichnungsmaterial kann wahlweise für die Anfertigung positiver oder negativer Reliefkopien einer Vorlage oder auch für deren Kombination verwendet werden.

Es ist bekannt, daß normalerweise positiv arbeitende Kopiermaterialien auf der Basis von 1,2-Benzochinon- und 1,2-Naphthochinon-(2)-diazid-sulfonsäurederivaten bei der Einhaltung einer bestimmten Folge von Verarbeitungsschritten durch eine Polaritätsumkehr auch negative Reliefbilder ergeben. Die Herstellung und Verarbeitung solcher Kopiermaterialien ist beispielsweise in den US-A 3 264 104, GB-A 2 082 339, DE-A 25 29 054, entsprechend US-A 4 104 070, DE-A 33 25 022, entsprechend US-A 45 81 321 und EP-A 0 212 482 beschrieben.

Die lichtempfindliche Schicht der bekannten Kopiermaterialien besteht im wesentlichen aus alkalilöslichen Kresol-Formaldehyd-Novolakharzen in Verbindung mit lichtempfindlichen Substanzen, wie 1,2-Benzo- oder 1,2-Naphthochinon-(2)-diazidderivaten. Harze und lichtempfindliche Verbindungen werden in einem organischen Lösemittel oder Lösemittelgemisch gelöst und in dünner Schicht auf einen für den jeweiligen Anwendungszweck geeigneten Schichttträger aufgebracht. Die Harzkomponenten dieser Kopierlackgemische sind zwar in wäßrig-alkalischen Lösungen löslich, doch üben die lichtempfindlichen 1,2-Chinondiazidverbindungen eine lösungshemmende Wirkung auf das Harz aus. Bei der bildmäßigen Belichtung des beschichteten Trägers mit aktinischer Strahlung erfährt die lichtempfindliche Verbindung durch die Bestrahlung eine strukturelle Umwandlung, wodurch die belichteten Bereiche der Beschichtung löslicher werden als die unbelichteten. Aufgrund dieses Löslichkeitsunterschieds lösen sich die belichteten Bereiche der Kopierschicht in einer alkalischen Entwicklerlösung, während die nicht belichteten Bereiche im wesentlichen unverändert erhalten bleiben, so daß auf dem Schichtträger ein positives Reliefbild der Vorlage entsteht.

In den meisten Fällen wird das belichtete und entwickelte Kopiermaterial noch mit einem Ätzmittel behandelt, wobei die vom Entwickler nicht abgelösten Bereiche der Kopierschicht den Schichtträger vor dem Ätzmittel schützen. Auf diese Weise wird auf dem Schichtträger ein Ätzbild erzeugt, das in seiner Polarität der beim Belichten verwendeten Maske, Schablone oder sonstigen Vorlage entspricht.

Bei einer speziellen Ausführung einer negativ arbeitenden Kopierschicht, z.B. einem Photoresist, auf der Basis von 1,2-Chinondiazidverbindungen werden nach Bildbelichtung und anschließender Wärmebehandlung die vom Licht getroffenen Bereiche der Schicht durch Vernetzung der Harzmoleküle "gehärtet". Die Härtung der Harzkomponenten erfolgt in der Regel durch einen in die Schicht eingebauten "Vernetzer", der durch die Säure, die bei der Belichtung des 1,2-Chinondiazids entsteht, und durch die Wärmebehandlung aktiviert wird. Die beim Erwärmen angewandten Temperaturen liegen unterhalb der Zersetzungstemperatur des 1,2-Chinondiazids. Das Erwärmen kann durch Bestrahlung, Konvektion, durch Kontakt mit erwärmten Flächen, z.B. Walzen, oder durch Tauchen in ein erwärmtes Bad einer inerten Flüssigkeit, z.B. Wasser, erfolgen. Die Temperatur kann zwischen 100 und 150 °C, vorzugsweise zwischen 90 und 130 °C liegen.

Wirksame Vernetzer sind im allgemeinen Verbindungen, die unter den beschriebenen Säure- und Temperaturverhältnissen leicht ein Carboniumion bilden können. Beispiele hierfür sind die Hexamethylolmelaminether gemäß DE-A 33 25 022, entsprechend US-PS 4 581 321, sowie die in der EP-A 0 212 482 vorgeschlagenen Verbindungen mit zwei oder mehreren Hydroxy- oder Alkoxymethylgruppen in aromatischen Molekülstrukturen, wie 1,4-Bis-hydroxymethylbenzol oder 4,4′-Bis-methoxymethyldiphenylether. Als Vernetzer ist auch 2,6-Dimethylol-p-kresol gemäß US-A 4 404 272 bekannt.

Nach der Wärmebehandlung wird die Photoresistschicht in der Regel einer Totalbelichtung ("Flutbelichtung") unterworfen, um die noch lichtempfindlichen Schichtbereiche vollständig in eine alkalilösliche Form umzuwandeln. Die Flutbelichtung kann im allgemeinen unter der gleichen Lichtquelle erfolgen, die auch für die Bildbelichtung verwendet wurde.

Im Anschluß an die Flutbelichtung wird mit einer wäßrigalkalischen Entwicklerlösung entwickelt, die üblicherweise auch bei einem positiv arbeitenden Photoresist verwendet wird, z.B. wäßrige Lösungen von Natriumhydroxyd, Tetramethylammoniumhydroxyd, Trimethylhydroxyethylammoniumhydroxid, Alkaliphosphaten, -silikaten oder -carbonaten, die Netzmittel oder kleinere Mengen organischer Lösemittel enthalten können. Bei der Entwicklung werden die Schichtbereiche ausgewaschen, die bei der ursprünglichen Bildbelichtung nicht vom Licht getroffen wurden (Negativ-Kopiermaterial).

Bei positiv arbeitenden Kopierschichten gleicher Schichtzusammensetzung in der durch geeignete Prozeßführung der Härtungsprozeß nicht in Gang gesetzt wurde, werden dagegen die bei der Bildbelichtung belichteten Schichtbereiche durch den Entwickler ausgewaschen (Positiv-Kopiermaterial).

In den meisten Fällen wird der belichtete und entwickelte Schichtträger noch mit einem Ätzmittel behandelt, wobei je nach Verarbeitungsverfahren, die auf dem Schichtträger zurückbleibende Kopierschicht schützt. Das Ätzmittel kann deshalb nur auf die schichtfreien Bereiche des Schichtträgers einwirken. Auf diese Weise wird auf dem Schichtträger ein Ätzbild erzeugt, das im Falle einer positiv arbeitenden Kopierschicht, der Polarität der Belichtungsmaske entspicht oder, im Falle einer negativ arbeitenden Kopierschicht, die Polarität der Belichtungsmaske umkehrt.

Das nach den oben beschriebenen Verarbeitungsverfahren auf dem Schichtträger erzeugte positive bzw. negative Reliefbild der Kopierschicht eignet sich für verschiedene Anwendungszwecke, z.B. als Belichtungsmaske oder als Bild bei der Herstellung von Halbleiterbauelementen in der Mikroelektronik, als Druckform für den Hoch-, Tief- oder Flachdruck sowie für die galvanische Herstellung von Nickelrotationszylindern.

Wichtige Kriterien, nach denen die Eignung einer Kopierschicht, z.B. eines Photoresists, für kommerzielle Zwecke beurteilt wird, sind unter anderem: die Lichtempfindlichkeit, der Entwicklungs-und Bildkontrast, die Resistauflösung und die Haftung des Resists auf dem Schichtträger.

Große Lichtempfindlichkeit ist für einen Photoresist insbesondere dann wichtig, wenn er für Anwendungen eingesetzt wird, bei denen mehrere Belichtungen erforderlich sind, z.B. bei der Erzeugung von Mehrfachbildern in einem Wiederholungsverfahren oder in solchen Fällen, wo Licht geringerer Intensität verwendet wird, z.B. bei Projektionsbelichtungsverfahren, bei denen das Licht durch eine Reihe von Linsen und monochromatischen Filtern geleitet wird, wie bei Projektionsbelichtungsgeräten ("Steppern"), die mit monochromatischem Licht arbeiten.

Der Entwicklungskontrast ist ein Maßstab für die Fähigkeit eines Photoresists, die Abmessungen der Maske zuverlässig und genau durch die gesamte Dicke der Schicht hindurch zu übertragen. Im Idealfall haben die Abmessungen an der Oberseite der Schicht genau die gleichen Abmessungen wie die an ihrer Unterseite. Ein Photoresist mit verbessertem Kontrast besitzt also steilere Flanken.

Die Resistauflösung betrifft das Vermögen eines Photoresistsystems, auch die feinsten Linien und Zwischenräume einer für die Belichtung verwendeten Maske wiederzugeben, wobei die belichteten und entwickelten Bereiche ein hohes Maß an Kantensteilheit und -schärfe aufweisen.

Auf vielen technischen Einsatzgebieten, insbesondere bei der Herstellung von Halbleiterelementen in der Mikroelektronik, muß der eingesetzte Photoresist ein besonders großes Auflösungsvermögen aufweisen, wenn er sehr kleine Linien- und Zwischenraumbreiten (ca. 1 μm) wiedergeben soll. Diese Eigenschaft, die Fähigkeit zur Wiedergabe kleinster Abmessungen in der Größenordnung von 1 μm und darunter, ist für die großtechnische Herstellung von integrierten Schaltungen auf Siliziumchips und ähnlichen Bauteilen von größter Bedeutung. Die Integrationsdichte auf einem solchen Chip kann bei Einsatz photographischer Verfahren nur durch eine Steigerung des Auflösungsvermögens des Photoresists verbessert werden. Die Miniaturisierung von Mikroprozessoren und anderen Halbleiterelementen in der Mikroelektronik macht es notwendig, für die Strukturierung geeigneter Substrate solche Verfahren einzusetzen, die möglichst schnell, sicher und einfach ablaufen und reproduzierbare Ergebnisse liefern.

In der Fach- und Patentliteratur werden Verfahren vorgeschlagen, die es ermöglichen, bei bestimmter Prozeßführung, mit Photoresistschichten auf der Basis von 1,2-Chinondiazidderivaten negative Resist-Reliefbilder herzutellen, wie S.A. MacDonald et al., "Image Reversal: The Production of a Negative Image in an Positive Photoresist", S. 114, IBM Research Disclosure, 1982; E. Alling et al., "Image Reversal of Positive Photoresist. A new Tool for Advancing Integrated Circuit Fabrication", Proceedings of the SPIE, Vol. 539, S. 194, 1985; US-A 4 104 070 und US-PS 4 576 901.

Die technische Durchführung dieser bekannten Bildumkehrverfahren ist teilweise sehr kompliziert und deshalb für die Photoresistverarbeitung in der Mikroelektronik nicht praktikabel. Die Qualität der Resiststrukturen ist in den meisten Fällen nicht sicher reproduzierbar. Nachteilig ist außerdem, daß für die Durchführung des Umkehrprozesses bei einigen Verfahren entweder eine verfahrenstechnisch nur schwierig zu beherrschende Aminbegasung der Photoresistschicht erforderlich ist oder in die Photoresistschicht Zusätze, die die Lagerstabilität deutlich vermindern, eingebaut werden müssen.

Bekannt ist auch, daß das Auflösungsvermögen eines Photoresists auf der Basis von 1,2-Chinondiazidderivaten bei einem Bildumkehrverfahren um ca. 0,2 - 0,3 μm besser ist als bei positiver Prozeßführung.

In der EP-A 0 212 482 wird ein Verfahren zur Herstellung negativer Relief-Bildstrukturen aus einem positiv arbeitenden Aufzeichnungsmaterial vorgeschlagen, das im wesentlichen ein wasserunlösliches, in alkalischen Lösemitteln lösliches Harz, einen 1,2-Chinon-(2)-diazid-4-sulfonsäureester als lichtempfindliche Verbindung und einen in Gegenwart von Säure wirkenden Vernetzer enthält. Bedingt durch die Zusammensetzung der Schicht und durch den Prozeßablauf bei der Herstellung negativer ReliefBildstrukturen werden einige der Nachteile der bekannten früheren Verfahren beseitigt, wie z.B. weniger Prozeßschritte, keine Behandlung mit

alkalisch reagierenden oder salzbildenden Stoffen, kein Einsatz besonders energiereicher Belichtungsquellen, wie z.B. Elektronenstrahlen. Von Nachteil ist jedoch, daß die in EP-A 0 212 482 vorgeschlagene lichtempfindliche Schicht vorrangig für die Belichtung im mittleren UV-Bereich (313 - 365 nm) und für die i-line-Belichtung (365 nm) geeignet ist.

Aus der japanischen Offenlegungsschrift 27 835/88 sind Arylester von halogensubstituierten Naphthochinondiazidsulfonsäuren bekannt, die für Photoresistmaterialien verwendet werden können. Sie sind grundsätzlich geeignet für Licht der Wellenlänge $\lambda$ = 365 nm und Breitbandbelichtung mit Quecksilberhochdrucklampen. Die Lichtempfindlichkeit bei 436 nm-Belichtung ist sehr gering und nicht praxisgerecht. Sie sind insbesondere geeignet für elektronenstrahlungsempfindliche Resistformulierungen, bei denen halogensubstituierte sich als besonders günstig erweisen.

Aus der CH-A 341 071 sind Diazooxoderivate von aromatischen Sulfonamiden bekannt, bei denen der Amidostickstoff durch einen cyclischen Terpenylrest von hohem Molekulargewicht substituiert ist. Dazu zählen auch 1,2-Naphthochinon-2-diazid-4,7-disulfonsäurediamide, bei denen die Amidostickstoffe in der angegebenen Weise substituiert sind. Die Verbindungen sind durch den großen Terpenylrest leicht löslich in einfachen organischen Lösemitteln, wie Alkylestern und Ketonen.

Für die 365 nm-Belichtung stehen nur wenige brauchbare Belichtungsgeräte (i-line-Stepper) zur Verfügung, da hierfür die technologischen Voraussetzungen noch nicht optimal gelöst sind. Im Gegensatz hierzu wird bei der mikrolithographischen Technik gegenwärtig ganz überwiegend mit g-line-Belichtung (436 nm) gearbeitet. Für diese Wellenlänge ist die überwiegende Mehrzahl der Belichtungsgeräte für Hochauflösung (g-line-Stepper) eingerichtet. Es besteht daher ein dringender Bedarf, ohne kapitalintensive Neuinstallation von Steppern, die bei kürzerer Wellenlänge arbeiten, das Auflösungspotential bei g-line-Belichtung zu verbessern. Dieses ist mit einem Bildumkehrverfahren möglich.

Der für i-line-Belichtung (365 nm) gefundene Lösungsweg ist jedoch für g-line-Belichtung (436 nm) nicht praktikabei. Die Lichtempfindlichkeit dieser bekannten Resists ist bei 436 nm sehr niedrig, was sich in längeren nicht praxisgerechten Belichtungszeiten und dem damit verbundenen schlechteren Entwicklungs- und Bildkontrast und geringeren Auflösungsvermögen von Mikrostrukturen bemerkbar macht.

Es besteht daher dringender Bedarf und es war deshalb Aufgabe der vorliegenden Erfindung bildumkehrfähige Photoresists zu schaffen, die bei 436 nm eine hinreichende Lichtempfindlichkeit aufweisen und welche die Nutzung der g-line-Stepper für die Herstellung einer weiteren Generation von Speicherbaulelementen mit 0,2 - 0,3 µm kleineren Dimensionen der Strukturen gestattet.

Für die Lösung dieser Aufgabe werden neue, strahlungsempfindliche Verbindungen vorgeschlagen, die Ester oder Amide einer 1,2-Naphthochinon-(2)-diazid-4-sulfonsäure der allgemeinen Formel

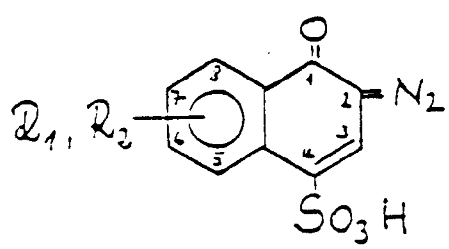

(I)

darstellen, worin

$R_1$ und $R_2$ gleich oder verschieden sind und Wasserstoff, Alkyl, eine Alkylether- oder Alkylthioethergruppe, deren Kohlenstoffketten durch Ethersauerstoffatome unterbrochen sein können, eine Acylamino-, Carbonsäureester-, Sulfonsäureester- oder Sulfonamidgruppe bedeuten, wobei

$R_1$ und $R_2$ nicht gleichzeitig Wasserstoff sind. Vorzugsweise werden Verbindungen vorgeschlagen nach der allgemeinen Formel I, worin

$R_1$ und $R_2$ mit der obengenannten Einschränkung gleich oder verschieden sind und Wasserstoff, Alkyl mit 1 bis 6 Kohlenstoffatomen, eine Alkylether- oder Alkylthioethergruppe, deren Kohlenstoffketten durch Ethersauerstoffatome unterbrochen sein können, mit insgesamt 1 bis 6 Kohlenstoffatomen, oder eine Acylaminogruppe mit 2 bis 6 Kohlenstoffatomen bedeuten. Insbesondere sind Verbindungen geeignet, worin

$R_1$ Wasserstoff und

$R_2$ Alkylether- oder Alkylthioethergruppen, deren Kohlenstoffkette durch Ethersauerstoffatome unterbrochen sein kann, mit 1 bis 6 Kohlenstoffatomen

bedeuten. Ganz besonders bewährt haben sich Verbindungen, worin

$R_1$ Wasserstoff oder Methyl und

$R_2$ Methyl oder eine Alkylethergruppe mit 1 bis 4 Kohlenstoffatomen
bedeuten. Spezielle Verbindungen sind solche, worin
$R_1$ in Position 6 Wasserstoff oder Methyl und
$R_2$ in Position 7 Methyl oder die Methylethergruppe bedeuten, ganz bevorzugt solche, worin
$R_1$ in Position 6 Wasserstoff und
$R_2$ in Position 7 die Methylethergruppe
bedeuten.

Durch die Erfindung wird erreicht, die Absorption der mit den erfindungsgemäßen Verbindungen herstellbaren strahlungsempfindlichen Ester oder Amide aus der 1,2-Naphthochinon-(2)-diazid-Reihe nach längeren Wellenlängen zu verschieben, wobei gleichzeitig die Bildung einer stärkeren Säure bei der Photoreaktion aufrechterhalten wird. Trotz der Verschiebung des Absorptionsmaximums zu größeren Wellenlängen weisen die erfindungsgemäßen Verbindungen jedoch auch bei i-line-Belichtung ($\lambda$=365 nm) noch hinreichend hohe Absorption auf. Die Uberlappung der Absorptionsbereiche der photoaktiven Verbindung mit dem Emissionsbereich der Strahlungsquelle ist zwar notwendige Voraussetzung für das Eintreten einer Photoreaktion, die Erfüllung dieser Bedingung allein reicht jedoch noch nicht aus, um die gewünschte Photoreaktion auch tatsächlich ablaufen zu lassen.

Überraschenderweise konnten jedoch Substitutionsmuster aus der Reihe der 1,2-Naphthochinon-(2)-diazid-4-sulfonsäurederivate gefunden werden, die bei einer sicheren und technisch praktikablen Arbeitsweise auch die Durchführung von negativ arbeitenden Bildumkehrprozessen mit hoher Effizienz gestatten.

Als Ester kommen die Reaktionsprodukte der 1,2-Naphthochinon(2)-diazid-4-sulfonsäuren I oder ihrer Chloride mit aliphatischen oder aromatischen Mono- oder Polyhydroxyverbindungen in Betracht. Bevorzugt sind die Reaktionsprodukte mit mono- oder polyfunktionellen Phenolderivaten der allgemeinen Formeln II, III und IV.

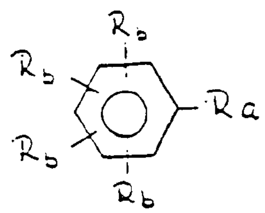

(II)

in der
$R_a$ = H, $-X-R_c$ oder

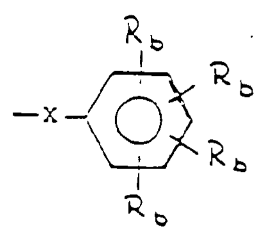

$R_b$ = H, eine OH-Gruppe, Halogen oder eine niedere Alkylgruppe bedeutet, wobei mindestens eine und höchstens sechs $R_b$-Gruppen OH-Gruppen sind,
X = eine -Kohlenstoff-Bindung, - O -, - S -, - $SO_2$-,

$$-\underset{O}{\underset{\parallel}{C}}-,\quad -\underset{O}{\underset{\parallel}{C}}-(CH_2)_n-,\quad -CH_2-\underset{O}{\underset{\parallel}{C}}-(CH_2)_n,$$

$$-\underset{O}{\underset{\parallel}{C}}-(CH_2)_n-\underset{O}{\underset{\parallel}{C}}-,\quad -\underset{O}{\underset{\parallel}{C}}-O-,\quad -\underset{O}{\underset{\parallel}{C}}-O-(CH_2)_n-,$$

$$-CH_2-\underset{\underset{O}{\|}}{C}-O-(CH_2)_n-,\quad -(CH_2)_p-,\quad CH_3-\overset{|}{\underset{|}{C}}-CH_3,$$

$$CH_3-\overset{|}{\underset{|}{C}}-(CH_2)_n-Y,\quad H-\overset{|}{\underset{|}{C}}-(CH_2)_n-Y$$

oder

$$CF_3-\overset{|}{\underset{|}{C}}-CF_3,$$

wobei jeweils einzelne $CH_2$-Gruppen durch Etherfunktionen oder der Wasserstoff durch Substituenten ersetzt sein können, darstellt und

n = 1 oder 2, und

P = 1 bis 3

Y = H oder eine gegebenenfalls substituierte Alkoxy-, Carboxy-, Carboxyalkyl-, Carboxyalkoxyalkyl- oder Arylgruppe und

$R_c$ = H oder eine gegebenenfalls substituierte Alkyl- oder Arylgruppe

bedeuten

oder der Formel III

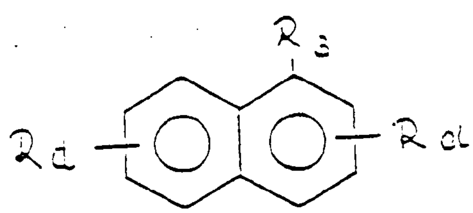

(III)

in der

$R_3$ = H oder

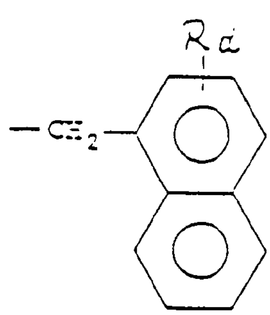

bedeutet und

$R_d$ = H oder eine OH-Gruppe darstellt, wobei mindestens eine der $R_d$-Gruppen eine OH-Gruppe ist

oder der Formel IV

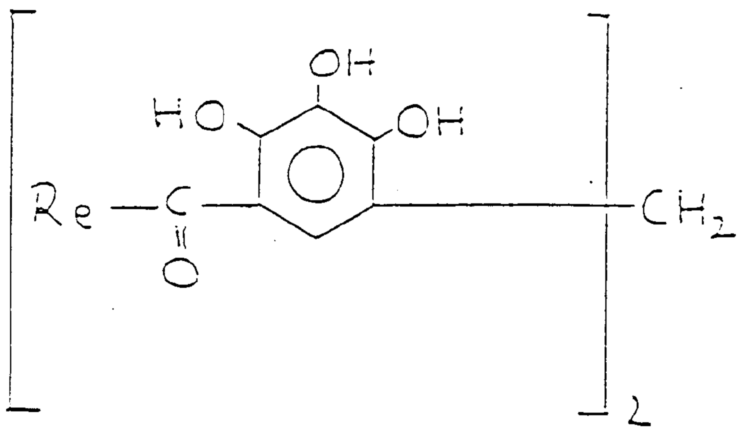

(IV)

in der

$R_e$ = H oder eine gegebenfalls substituierte Alkyl-, Alkoxy- oder Arylgruppe bedeutet

oder der Formel V

$$R_f - OH \qquad (V)$$

in der

$R_f$ = eine geradkettige oder verzweigte, ggf. durch Halogen oder einen Acylaminorest substituierte Alkyl-, Cycloalkyl- oder Aralkylgruppe mit 1 bis 14 C-Atomen, deren Kohlenstoffkette durch Ethersauerstoffatome unterbrochen sein kann, oder der Gruppe - Z - OH

in der

Z = ein Alkylenrest mit 2 bis 12 C-Atomen oder ein Cycloalkylenrest mit 8 bis 18 C-Atomen, deren C-Ketten durch Ethersauerstoffatome unterbrochen sein können, oder ein Aralkylenrest mit 8 bis 16 C-Atomen, wobei die cycloaliphatischen und aromatischen Glieder durch eine Einfachbindung, durch - O -, - S -, - $SO_2$ -, - CO -

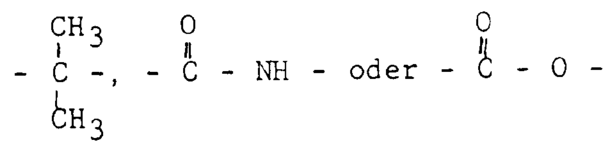

oder

verbunden sein können,

bedeuten.

Zu den bevorzugten Phenolderivaten zählen beispielsweise hydroxylgruppentragende Benzolverbindungen, wie 1,2-Dihydroxybenzol, 1,3-Dihydroxybenzol, 1,4-Dihydroxybenzol, 1,2,3-Trihydroxybenzol, 1,2,-4-Trihydroxybenzol, 1,3,5-Tri-hydroxybenzol usw.;

Dihydroxybenzophenone, wie 2,2′-Dihydroxybenzophenon, 2,3′-Dihydroxybenzophenon, 2,4-Dihydroxybenzophenon, 2,4′-Dihydroxybenzophenon, 2,5-Dihydroxybenzophenon, 3,3′-Dihydroxybenzophenon, 4,4′-Dihydroxybenzophenon usw.;

Trihydroxybenzophenone, wie 2,2′,6-Trihydroxybenzophenon, 2,3,4-Trihydroxybenzophenon, 2,4,4′-Trihydroxybenzophenon, 2,4,6-Trihydroxybenzophenon, 3,4,5-Trihydroxybenzophenon, usw.;

Tetrahydroxybenzophenone, wie z.B. 2,2′,3,4-Tetrahydroxybenzophenon, 2,2′,4,4′-Tetrahydroxybenzophenon, 2,2′,4,6′-Tetrahydroxybenzophenon, 2,2′,5,6′-Tetrahydroxybenzophenon, 2,3′,4,4′-Tetrahydroxybenzophenon, 2,3′,4,6-Tetrahydroxybenzophenon, 2,4,4′,6-Tetrahydroxybenzophenon, 3,3′,4,4′-Tetrahydroxybenzophenon usw.;

Pentahydroxybenzophenone; Hexahydroxybenzophenone;

Dihydroxy- und Trihydroxy-phenyl-alkyl-ketone, wie 2,4-Dihydroxyphenyl-alkyl-ketone, 2,5-Dihydroxyphenyl-alkyl-ketone, 3,4-Dihydroxyphenyl-alkyl-ketone, 3,5-Dihydroxyphenyl-alkyl-ketone, 2,3,4-Trihydroxyphenyl-alkyl-ketone, 3,4,5-Trihydroxyphenyl-alkyl-ketone, 2,4,6-Trihydroxyphenyl-alkyl-ketone usw.; wobei vorzugsweise Alkylgruppen mit 1 bis 12 C-Atomen, die gegebenenfalls verzweigt sind, eingesetzt werden, wie z.B. Methyl, Ethyl, Butyl, n-Hexyl, Heptyl, Decyl, Dodecyl usw.;

Dihydroxyphenyl-aralkyl-ketone; Trihydroxyphenyl-aralkylketone; Dihydroxydiphenyle; Trihydroxydiphenyle, wie 2,2′,4-Trihydroxydiphenyl; Tetrahydroxydiphenyl, wie z.B. 2,2′,4,4′-Tetrahydroxydiphenyl; Dihydroxydiphenylether; Dihydroxydibenzylether; Dihydroxydiphenylalkane, vorzugsweise mit niederen Alkylenketten, wie z.B. Methylen, Ethylen, Propylen usw.; Dihydroxybenzoesäure; Trihydroxybenzoesäuren; Dihydroxy- und Trihydroxy-benzoesäurealkylester, wobei die Alkylgruppe vorzugsweise 1 bis 12 C-Atome, insbesondere 1 bis 8 C-Atome besitzt, wie z.B. n-Butyl-2,4-, -2,5-, -3,4- und -3,5-dihydroxybenzoat, 2,4,4-Trimethylpentyl-2,4-dihydroxybenzoat usw.; Dihydroxy- und Trihydroxybenzoesäurephenylester; Dihydroxy-, Trihydroxy- und Tetrahydroxydiphenylsulfide, wie z.B. 4,4′-Dihydroxydiphenylsulfid; Dihydroxydiphenylsulfone; Dihydroxy- und Trihydroxyphenylnaphthylketone, wie z.B. 2,3,4-Trihydroxyphenylnaphthyl-1-keton und ähnliche Verbindungen.

Zu den Verbindungen der allgemeinen Formel (II), bei denen wenigstens eine $R_b$-Gruppe ein Halogenatom oder eine niedere Alkylgruppe darstellt, gehören beispielsweise 2,4-Dihydroxy-3,5-dibrombenzophenon, 5-Brom-2,4-dihydroxybenzoesäure und ihre Ester, 2,4,2′,4′-Tetrahydroxy-3,5,3′,5′-tetrabromdiphenyl, 4,4′-Dihydroxy-2,2′-dimethyl-5,5′-di-tert.-butyldiphenyl, 4,4′-Dihydroxy-2,2′-dimethyl-5,5′-di-tert.-butyldiphenyl-sulfid, 2,4,2′,4′-Tetrahydroxy-3,5,3′,5′-tetrabromdiphenylsulfon und ähnliche Verbindungen.

Die Hydroxygruppen enthaltenden Benzophenone, insbesondere die Trihydroxybenzophenone, werden als Phenolverbindungen der allgemeinen Formel (II) bevorzugt eingesetzt.

Zu den Phenolverbindungen der allgemeinen Formel (III) gehören vorzugsweise folgende Verbindungen: Dihydroxynaphthaline, wie 1,2-Dihydroxynaphthalin, 1,4-Dihydroxynaphthalin, 1,5-Dihydroxynaphthalin, 1,6-Dihydroxynaphthalin, 1,7-Dihydroxynaphthalin, 1,8-Dihydroxynaphthalin, 2,3-Dihydroxynaphthalin, 2,6-Dihydroxynaphthalin, 2,7-Dihydroxynaphthalin usw.; Dihydroxydinaphthylmethane, wie 2,2′-Dihydroxydinaphthylmethan usw.

Die Dihydroxynaphthaline werden bevorzugt eingesetzt. Die Hydroxylgruppen der Dihydroxynaphthaline können sich dabei sowohl an einem Kern, als auch an beiden Kernen des Naphthalinmoleküls befinden.

Zu den Phenolverbindungen der allgemeinen Formel (IV) gehören vorzugsweise folgende Verbindungen: Bis-(3-benzoyl-4,5,6-trihydroxyphenyl)-methan, Bis-(3-acetyl-4,5,6-trihydroxyphenyl)-methan, Bis-(3-propionyl-4,5,6-trihydroxyphenyl)-methan, Bis-(3-butyryl-4,5,6-trihydroxyphenyl)-methan, Bis-(3-hexanoyl-4,5,6-trihydroxyphenyl)-methan, Bis-(3-heptanoyl-4,5,6-trihydroxyphenyl)-methan, Bis-(3-decanoyl-4,5,6-trihydroxyphenyl)-methan, Bis-(3-octadecanoyl-4,5,6-trihydroxyphenyl)-methan usw.

Als Ester kommen außerdem die Reaktionsprodukte der 1,2-Naphthochinon-(2)-diazid-4-sulfonsäuren I oder deren Chloride mit ein- oder zweiwertigen Alkoholen der allgemeinen Formel V in Betracht.

Zu den ein- und zweiwertigen Alkoholen der allgemeinen Formel V sind vorzugsweise zu zählen, z.B. n-Butylalkohol, sek.-Butylalkohol, n-Amylalkohol, n-Octylalkohol, n-Dodecylalkohol, Laurylalkohol, Myristylalkohol, Cyclohexanol, Benzylalkohol, β-Phenethylalkohol, Di-, Tri-, Tetraethylenglykol, 1,4-Dihydroxycyclohexan.

Als Amide können die Reaktionsprodukte aus den 1,2-Naphthochinon-(2)-diazid-4-sulfonsäuren I oder deren Chloride mit aromatischen oder aliphatischen primären oder sekundären Aminen der allgemeinen Formel VI eingesetzt werden,

$$H - NR_gR_h \qquad (VI)$$

in der

$R_g$ = Wasserstoff, ein ggf. substituierter Alkyl-, Cycloalkyl-, Aryl- oder Aralkylrest mit 1 bis 14 C-Atomen, dessen C-Kette durch Ethersauerstoffatome unterbrochen sein kann,

$R_h$ = $- E - NR_gH$

worin

E = ein Alkylenrest mit 2 bis 12 C-Atomen, dessen C-Kette durch Ethersauerstoffatome unterbrochen sein kann, ein Cycloalkylen-, Arylen- oder Aralkylenrest mit 8 bis 18 C-Atomen, wobei im Falle mehrkerniger Verbindungen die cycloaliphatischen und aromatischen Glieder durch eine Einfachbindung, durch - O -, - S -, - $SO_2$ -, - CO - oder - $R_g$ - verbunden sein können,

bedeuten.

Vorzugsweise sind es längerkettige aliphatische Amine, deren C- Kette durch O-Atome unterbrochen sein kann, oder cyclische oder aromatische Amine, wie n-Hexylamin, Laurylamin, Cyclohexylamin, Cyclododecylamin, o-, p-, m-Toluidin, o-, p-Anisidin, p-Phenetidin, 1,6-Diamino-(n)-hexan, 1,10-Diamino-(n)-decan, o-, p-, m-Phenylendiamin, 4,4′-Diaminodiphenylether, 4,4′-Bis(aminomethyl)-diphenylether, 1,4-Bis(aminomethyl)-benzol, 1,4-Bis(propylaminomethyl)-diphenylether, 4,4′-Bis(sek.-butylaminomethyl)-diphenylether, 1,4-Bis(n-octylamino)-cyclohexan, 1,4-Bis(sek.-butylamino)-cyclohexan, 4,4′-Bis(2-methoxyethylaminomethyl)-diphenylether, 4,4′-Bis(2-phenylethylaminomethyl)-diphenylether.

Die Herstellung von strahlungsempfindlichen Estern aus der Reihe der Naphthochinondiazide ist bekannt und beispielhaft beschrieben in US-A 3 046 118, 4 397 937 und EP-A 0 140 273, die von strahlungsempfindlichen Amiden in DE-C 865 410. Die strahlungsempfindlichen Ester und Amide gemäß der Erfindung werden analog dieser Verfahren aus den Chloriden der 1,2-Naphthochinon-(2)-diazid-4-sulfonsäuren I durch Umsetzung mit einer Hydroxyverbindung des Typs II bis V oder einer Aminverbindung des Typs VI in der Regel in Gegenwart eines Säureakzeptors hergestellt.

Geeignete Lösemittel für die Durchführung der Reaktion sind: Aceton, Tetrahydrofuran, Methylenchlorid, Pyridin u.a. Der Säureakzeptor kann anorganisch sein, wie z.B. Natriumcarbonat, oder organisch, wie ein Natriumsalz einer schwachen organischen Säure, ein tertiäres Amin, z.B. Triethylamin oder N-Methylmorpholin.

Die auf diese Weise erhaltenen strahlungsempfindlichen Verbindungen können gegebenenfalls noch gereinigt werden.

Die 1,2-Naphthochinon-(2)-diazid-4-sulfonsäuren der allgemeinen Formel I, die als Ausgangsstoffe für die Herstellung der strahlungsempfindlichen Ester und Amide gemäß der Erfindung verwendet werden, sind in der Tabelle 1 des Anhangs aufgeführt. Ihre Herstellung verläuft über mehrere Zwischenstufen, die zum Teil in der Literatur[1)] beschrieben sind oder analog literaturbekannter Verfahren synthesiert wurden.

So erhält man beispielsweise 6-Methoxy- und 7-Methoxy-1,2-naphthochinon-(2)-diazid-4-sulfonsäure aus handelsüblichem 1-Acetylamino-6-naphthol bzw. 1-Acetylamino-7-naphthol (BAYER AG) und 8-Methoxy-1,2-naphthochinon-(2)-diazid-4-sulfonsäure aus handelsüblicher 1-Amino-8-naphthol-4-sulfonsäure (BAYER AG) in jeweils 7-stufigen Reaktionsfolgen.

Die Erfindung betrifft auch ein strahlungsempfindliches Gemisch, das ein in Wasser unlösliches, in wäßrig-alkalischen oder organischen Lösemitteln lösliches, zumindest aber quellbares harzartiges Bindemittel, gegebenenfalls einen Vernetzer sowie eine strahlungsempfindliche Verbindung oder ein Gemisch strahlungsempfindlicher Verbindungen enthält, wobei mindestens eine der strahlungsempfindlichen Verbindungen ein Ester oder ein Amid nach einem der Ansprüche 1 bis 8 ist.

Die Herstellung des erfindungsgemäß strahlungsempfindlichen Gemisches insbesondere von Photoresists, erfolgt durch Mischen der strahlungsempfindlichen Verbindungen mit den alkalilöslichen Bindemitteln, Lösemitteln und gegebenenfalls weiteren Zusätzen. Geeignete alkalilösliche Bindemittel sind beispielsweise Phenol- und Kresolharz-Novolake, Polyvinylphenol- oder Polyvinylalkylphenol-Harze.

Die alkali-löslichen Novolake und Polyvinylphenolharze, die für die Herstellung von lichtempfindlichen Gemischen verwendet werden können, sind bekannt. Ein Verfahren zur Herstellung solcher Novolake ist in "Chemistry and Application of Phenolic Resins" (Chemie und Anwendung von Phenolharzen), von A. Knop und W. Scheib, Springer Verlag, New York, 1979, Kapitel 4, beschrieben. Die Verwendung von Polyvinylphenolen ist z.B. aus US-A 3 869 292 bekannt.

Für die Herstellung des erfindungsgemäßen Gemisches werden der Novolak oder das Polyvinylphenol und die strahlungsempfindliche Verbindung in einem Lösemittel gelöst. Hierfür geeignete Lösemittel sind beispielsweise Glykolether, wie Ethylenglykolmonomethylether und Ethylenglykolmonoethylether oder auch deren Acetate, etwa Propylenglykolmethyletheracetat; Ester, wie Ethylacetat und Butylacetat; Ketone, wie Methylethylketon, Cyclopentanon und Cyclohexanon; sowie aromatische Kohlenwasserstoffe, wie Toluol und Xylol. Es können auch Gemische aus diesen Lösemitteln eingesetzt werden. Der Einsatz des Lösemittels bzw. Lösemittelgemisches hängt im Einzelfall unter anderem ab von dem jeweiligen Beschichtungsverfahren, der gewünschten Schichtdicke, den Trocknungsbedingungen, der Löslichkeit der einzelnen Komponenten und der Verdunstungsgeschwindigkeit des Lösemittels nach der Beschichtung des Schichtträgers mit dem Photoresistgemisch.

Dem erfindungsgemäßen strahlungsempfindlichen Gemisch können vor dem Aufbringen auf einen Schichtträger noch Zusätze, wie z.B. Farbmittel, Farbstoffe, Verlaufmittel, Weichmacher, Haftvermittler, Entwicklungsbeschleuniger, Tenside, wie nicht-ionische Tenside, und Vernetzungsmittel, zugegeben werden.

In bevorzugter Ausführung liegt der Gehalt an festen Bestandteilen des strahlungsempfindlichen Gemisches für das alkali-lösliche Bindemittel bei etwa 15 bei 99 Gewichtsprozent, für die strahlungsempfindliche Verbindung bei etwa 1 bis 85 Gewichtsprozent. Insbesondere ist das Bindemittel in einem Anteil von etwa 50 bis 97 Gewichtsprozent, und ganz besonders bevorzugt, von etwa 65 bis 93 Gewichtsprozent, bezogen auf das Gewicht der festen Bestandteile, in dem Gemisch enthalten. Der Anteil an strahlungsempfindlicher Ver-

Lit[1)] 1) H.E. Fierz-David u.a., Helv.chim.acta 30, 831 ff. (1947)
2) W. Huber, Helv.chim.acta 15, 1372 (1932)
3) H.E. Fierz-David, "Grundlegende Operationen der Farbenchemie", 7. Aufl., S. 172-175 (1947) und S. 251-252
4) H.T. Bucherer, J.pr.Chemie 1904 (2), 69, 49 ff.
5) Houben-Weyl, Bd. 6/I c, 241 ff. und Bd. 11/1, 457 ff.
6) Witt u. Kaufmann, Ber. 1891, 24, 3157 ff.
7) Anilin- und Extrakt-Fabriken, Basel, Chem. Zentralblatt 77, 474-477 (1906)
8) J. Schmidt u. W. Maier, Ber. 1931, 64, 767 ff.

bindung liegt insbesondere bei etwa 3 bis 50 Gewichtsprozent und ganz besonders bevorzugt bei etwa 7 bis 35 Gewichtsprozent, bezogen auf das Gewicht der festen Bestandteile des Gemisches. Zur Herstellung des Gemisches werden das Bindemittel und die strahlungsempfindliche Verbindung so mit dem Lösemittel gemisch, daß das Lösemittel in einem Anteil von 40 bis 90, vorzugsweise von etwa 60 bis 85 Gewichtsprozent, und insbesondere von etwa 65 bis 80 Gewichtsprozent, jeweils bezogen auf das Gewicht des gesamten Gemisches, vorhanden ist.

Farbstoffe, die als Zusätze für die erfindungsgemäßen Gemische verwendet werden können, sind zum Beispiel Methylviolett 2B (C.I. 42 535), Kristallviolett (C.I. 42 555), Malachitgrün (C.I. 42 000), Victoriablau B (C.I. 44 045) und Neutralrot (C.I. 50 040), sowie Cumarinfarbstoffe und Farbstoffe entsprechend der deutschen Patentanmeldung Aktenzeichen P 37 35 852.9. Diese Farbstoffe werden in einer Menge von 1 bis 10 Gewichtsprozent, bezogen auf die festen Bestandteile des Gemisches zugesetzt. Der Farbstoffzusatz verringert die Rückstreuung von Licht vom Schichtträger und trägt so zu einer verbesserten Auflösung bei.

Verlaufmittel können in einer Menge bis zu 5 Gewichtsprozent, bezogen auf das gemeinsame Gewicht von Bindemittel und strahlungsempfindlicher Verbindung, eingesetzt werden.

Geeignete Weichmacher sind zum Beispiel Phophorsäuretri-(β-chlorethyl)-ester, Stearinsäure, Dicampher, Polypropylen, Acetalharze, Phenoxyharze und Alkydharze, die in Anteilen von 1 bis 10 Gewichtsprozent, bezogen auf das gemeinsame Gewicht von Bindemittel und strahlungsempfindlicher Verbindung, zugesetzt werden können. Der Weichmacherzusatz verbessert die Beschichtungseigenschaften des Gemisches und ermöglicht das Auftragen in einer glatten und gleichmäßig dicken Schicht auf den Schichtträger.

Geeignete Haftvermittler zur besseren Haftung des Gemisches auf dem Schichtträger sind spezielle Siliciumorganische Verbindungen in einem Anteil bis zu 4 Gewichtsprozent, bezogen auf das gemeinsame Gewicht von Bindemittel und strahlungsempfindlicher Verbindung.

Als Entwicklungsbeschleuniger können beispielsweise aromatische Carbonsäuren oder Polyhydroxyverbindungen in einem Anteil bis zu 20 Gewichtsprozent, bezogen auf das gemeinsame Gewicht von Bindemittel und strahlungsempfindlicher Verbindung, zugegeben werden. Diese Beschleuniger führen dazu, daß die Löslichkeit der strahlungsempfindlichen Schicht sowohl in den belichteten als auch in den unbelichteten Bereichen zunimmt. Sie werden deshalb bei solchen Anwendungen eingesetzt, bei denen es in erster Linie auf die Entwicklungsgeschwindigkeit ankommt. Während die belichteten Bereiche der Photoresistschicht durch den Zusatz von Beschleunigern vom Entwickler schneller gelöst werden, bewirken die Entwicklungsbeschleuniger gleichzeitig aber auch einen größeren Verlust an Photoresistschicht aus den unbelichteten Bereichen, so daß eventuell ein gewisser Grad an Kontrast verloren geht.

Als nicht-ionische Tenside können zum Beispiel Nonylphenoxypoly(ethylenoxy)-ethanol, Octylphenoxy (ethylenoxy)-ethanol, und Dinonylphenoxy-poly(ethylenoxy)-ethanol in einem Anteil bis zu 10 Gewichtsprozent, bezogen auf das gemeinsame Gewicht vom Bindemittel und strahlungsempfindlicher Verbindung, verwendet werden.

Geeignete Vernetzungsmittel, die im allgemeinen einem strahlungsempfindlichen Gemisch zugesetzt werden, das für einen negativ arbeitenden Photoresist vorgesehen ist, sind in EP-A 0 212 482 und US-A 4 404 272 beschrieben. Es sind hauptsächlich Verbindungen aus der aromatischen Reihe mit zwei oder mehreren Hydroxy- oder Alkoxymethylgruppen im Molekül, wie 1,2-Bis-hydroxymethyl-benzol, 1,4-Bis-methoxymethylbenzol, 2,5-Bis-(hydroxymethyl)-furan, 2,6-Bis-hydroxymethyl-4-methyl-anisol, 1,4-Bis-(α-hydroxybenzyl)-benzol, 2,5-Dimethyl-1,4-bis-hydroxymethyl-benzol, 4,4′-Bis-hydroxymethyl-diphenylether, 4,4′-Bis-methoxydiphenyl, 2,6-Bishydroxymethyl-4-methoxy- oder -4-ethoxyphenol, 4,4′-Bis-methoxymethyl-diphenylether und Epoxykresol-Novolakharze, sowie auch Alkoxymethylmelaminderivate. Besonders bevorzugt sind: 2,6-Bishydroxymethyl-4-methyl-, -4-ethyl-, -4-propyl-, -4-isopropyl-, -4-n-butyl-, -4-tert.-butyl-, -4-(2-phenyl-2-propyl)-phenol. Die Vernetzungsmittel können in Mengen von 1,5 bis 20 Gewichtsprozent, bevorzugt von 3 bis 12 Gewichtsprozent bezogen auf den Feststoffgehalt eingesetzt werden.

In Anwesenheit von Säure und bei erhöhter Temperatur werden durch diese Verbindungen die Bindemittelmoleküle vernetzt, d.h. die strahlungsempfindliche Schicht wird gehärtet. Die für die Schichthärtung erforderliche Säure entsteht infolge der Photoreaktion der strahlungsempfindlichen Verbindung.

Erfindungsgemäß wird ferner ein strahlungsempfindliches Aufzeichnungsmaterial vorgeschlagen, bestehend aus dem oben beschriebenen im wesentlichen von Lösemitteln befreiten strahlungsempfindlichen Gemisch und einem Schichtträger.

Das strahlungsempfindliche Gemisch kann nach einem der in der Photoresistschicht üblichen Verfahren, wie Tauchen, Sprühen und Aufschleudern, auf den Schichtträger aufgebracht werden. Beim Aufschleudern kann zum Beispiel der Prozentanteil an Feststoffen in der Resistlösung so eingestellt werden, daß sich, in Abhängigkeit von der im Einzelfall verwendeten Aufschleudervorrichtung und der für den Aufschleudervorgang angesetzten Zeitspanne, eine Beschichtung in der gewünschten Dicke ergibt. Beispielhaft geeignete Trägermaterialien sind: Silicium, Aluminium, polymere Harze, Siliciumdioxid, dotiertes Siliciumdioxid, Siliciumnitrid,

Tantal, Kupfer, Polysilicium, Keramik und Aluminium/Kupfer-Legierungen.

Die nach dem beschriebenen Verfahren hergestellten strahlungsempfindlichen Gemisch eignen sich insbesondere zum Auftragen auf Silicium-Wafern, die eine Schicht aus Siliciumdioxid tragen, wie sie bei der Herstellung von Mikroprozessoren und anderen in der Mikroelektronik verwendeten Halbleiterbauelementen verwendet werden. Ebenso kann auch ein Wafer aus Silicium auf Aluminiumoxid eingesetzt werden. Der Schichtträger kann auch aus verschiedenen polymeren Harzen, insbesondere aus transparenten Polymeren, wie Polyestern, bestehen. Im Falle, daß der Schichtträger ein Silicium-oder Galliumarsenid-Wafer ist, ist vorzugsweise zwischen dem halbleitenden Material und der strahlungsempfindlichen Schicht noch mindestens eine weitere Schicht aufgebracht.

Nach dem Auftragen des strahlungsempfindlichen Gemisches auf den Schichtträger in Form eines Wafers wird das Ganze einer Vortrocknung bei etwa 80 bis 105 °C unterworfen. Diese Wärmebehandlung wird so lange fortgesetzt, bis das Lösemittel im wesentlichen verdampft ist und eine dünne Schicht von etwa 1 µm auf dem Schichtträger zurückbleibt.

Der beschichtete Träger wird anschließend in bekannter Weise mittels aktinischer Strahlung, insbesondere UV-Licht, z.B. der Wellenlänge $\lambda$=248 oder 365 nm oder kurzwelligem sichtbarem Licht, z.B. der Wellenlänge $\lambda$=405 oder 436 nm, durch geeignete Masken, Negative, Schablonen usw. bildmäßig bestrahlt oder der gerichteten Elektronenstrahlung oder der Strahlung eines Lasers ausgesetzt. Der Begriff aktinische Strahlung umfaßt sowohl den Röntgenbereich wie elektromagnetische Strahlung im technisch anwendbaren Wellenlängenbereich von =193-450 nm und Elektronenstrahlung.

Zur Entwicklung werden die bildmäßig belichteten Aufzeichnungsmaterialien in eine wäßrig-alkalische Entwicklerlösung getaucht, wobei man die Lösung vorzugsweise durch Durchblasen von Stickstoff stark bewegt. In der Technik werden häufig Sprühentwicklungsprozesse eingesetzt oder die Entwicklerlösung wird portionsweise auf den Wafer aufgebracht (Puddle-Entwicklung).

Nach dem Herausnehmen aus der Entwicklerlösung kann man das Material einer Wärmenachbehandlung bzw. Einbrennbehandlung zuführen, um die Haftung und die chemische Beständigkeit der Schicht gegen Ätzlösungen und andere Substanzen zu erhöhen. Die Wärmebehandlung nach dem Entwickeln kann aus einer Ofenhärtung von Schicht und Träger bei Temperaturen unterhalb des Erweichungspunktes der Schicht bestehen.

Für industrielle Anwendungen, insbesondere bei der Herstellung von Halbleiterbaulelementen auf Siliciumträgern mit einer Siliciumdioxidschicht, können die entwickelten Träger mit einer gepufferten Ätzlösung auf der Basis von Flußsäure behandelt werden. Die erfindungsgemäßen Photoresistgemische sind gegen derartige Ätzlösungen auf Säurebasis resistent und gewährleisten einen wirksamen Schutz der unbelichteten, mit dem Photoresistgemisch beschichteten Stellen des Trägers.

Die erfindungsgemäßen strahlungsempfindlichen Gemische eignen sich bevorzugt als Photoresistgemische. Sie können aber auch als strahlungsempfindliche Gemische für die Druckplattenherstellung oder für Farbprüffolien Verwendung finden.

Wird das Aufzeichnungsmaterial, das durch Beschichten eines Schichtträgers mit dem erfindungsgemäßen strahlungsempfindlichen Gemisch hergestellt wurde, für den negativ arbeitenden Bildumkehrprozeß eingesetzt, so wird nach der bildmäßigen Belichtung mit aktinischer Strahlung eine zweite kurzzeitige Wärmebehandlung bei einer Temperatur im Bereich zwischen 90 und 150 °C durchgeführt. Während dieser Wärmebehandlung erfolgt in den belichteten Bereichen der Photoresistschicht die Härtung des Bindemittels. Die Dauer der Wärmebehandlung liegt zwischen 10 und 90 Sekunden. Nach der Wärmebehandlung und Abkühlung des Aufzeichnungsmaterials wird die Photoresistschicht entwickelt oder vorzugsweise vor der Entwicklung einer ganzflächigen UV-Bestrahlung ausgesetzt (Flutbelichtung). Als Entwickler dienen wäßrig-alkalische Lösungen, die anorganische oder quaternäre organische Basen enthalten, gepuffert sein oder zusätzliche Additive (u.a. Netzmittel) enthalten können. Hierbei werden die Schichtbereiche, die bei der ursprünglichen Bebilderung von der Strahlung nicht getroffen wurden, ausgewaschen und die Schichtbereiche, die von der Strahlung getroffen wurden, nicht angegriffen (Negativ-Prozeß). Es entsteht eine Relief-Kopie der Belichtungsmaske mit geänderter Polarität (Negativ-Kopie).

Positiv-Prozeß und Bildumkehrprozeß können auch auf einem Schichtträger kombiniert werden (Photocomposing).

Herstellung der lichtempfindlichen Verbindungen

In den folgenden Beispielen werden Verfahren zur Herstellung der erfindungsgemäßen Verbindungen sowie deren Verwendung in strahlungsempfindlichen Gemischen und Aufzeichnungsmaterialien näher beschrieben.

Die Herstellung der erfindungsgemäßen Verbindungen aus den 1,2-Naphthochinon-(2)-diazid-4-sulfonsäuren I und den mono- bzw. polyfunktionellen Hydroxy- oder Aminoverbindungen II - VI gelingt in 9 bzw. 10 Reaktionsstufen.

Eine Anzahl der neuen Ester und Amide der 1,2-Naphthochinon-(2)-diazid-4-sulfonsäuren I (Tabelle 1), die synthetisiert und in Gemischen als strahlungsempfindliche Komponente erprobt wurden, sind in der Tabelle 2 des Anhangs aufgeführt. Die Numerierung der Verbindungen von 1 bis 19 ist in den Anwendungsbeispielen beibehalten. Im Beispiel 15 sind (in Verbindung mit Tabellen 4 und 5) die molaren Extinktionskoeffizienten ε und die "normierten" molaren Extinktionskoeffizienten ε′ einer Anzahl der in Tabelle 2 aufgeführten Verbindungen angegeben. Die Numerierung ist identisch mit der in Tabelle 2. Prozent- und Mengenverhältnisse sind, wenn nicht anders angegeben, in Gewichtseinheiten zu verstehen. Gewichtsteile (Gt) und Volumenteile (Vt) stehen im Verhältnis von g zu ml.

Synthesebeispiel 1

Synthese des Arylesters aus 2,3,4-Trihydroxybenzophenon und 7-Methoxy-1,2-naphthochinon-(2)-diazid-4-sulfonsäure

7-Methoxy-1-acetylaminonaphthalin

(1. Reaktionsstufe)

96 g Natriumhydroxid-Plätzchen werden in 1100 ml Wasser gelöst und in diese Lösung anschließend bei 20 - 25 °C unter Rühren 300 g 1-Acetylamino-7-naphthol (95 %ig) eingetragen. In die entstandene braune Lösung werden innerhalb 1 Stunde 480 g Dimethylsulfat eingetropft, wobei die Innentemperatur auf 35 - 40 °C ansteigt und das Reaktionsprodukt in feinkristalliner Form ausfällt. Das Reaktionsgemisch wird 1 Stunde bei 20 - 25 °C nachgerührt, anschließend 3o Minuten bei 90 - 100 °C Innentemperatur gehalten, dann abgekühlt, das fast farblose 7-Methoxy-1-acetylaminonaphthalin abgesaugt und an der Luft bei 20 - 25 °C getrocknet.
Ausbeute: 296 g (92 % d.Th.)
Fp.: 157 - 159 °C.

7-Methoxy-1-aminonaphthalin-hydrogensulfat

(2. Reaktionsstufe)

145 g 7-Methoxy-1-acetylaminonaphthalin werden unter Rühren in 670 ml 20 %iger Schwefelsäure eingetragen und die Suspension langsam unter Rückfluß zum Sieden erhitzt. Nach Erreichen der Siedetemperatur (100 - 103 °C) wird noch 3 Stunden lang diese Temperatur gehalten, wobei eine dunkelbraume Lösung entsteht. Anschließend wird von wenig öligen, dunklen Bestandteilen abfiltriert und in das noch warme Filtrat 85 g Natriumhydrogensulfat eingerührt. Man läßt auf 10 °C abkühlen und hält dann die Lösung noch weitere 2 - 3 Stunden bei 0 °C. Die kristalline Ausfällung wird abgesaugt und auf der Nutsche gut abgepreßt.

Das nutschenfeuchte 7-Methoxy-1-aminonaphthalin-hydrogensulfat wird in 650 ml Wasser bei 90 - 95 °C gelöst, zur Aufhellung der braunen Lösung 10 g Natriumdithionit und 35 g Aktivkohle zugesetzt, die heiße Lösung filtriert und das fast farblose Filtrat auf 0 °C abgekühlt. Nach 2 - 3 Stunden wird das ausgefallene farblose Produkt abgesaugt, auf der Nutsche mit Eiswasser gewaschen und gut abgepreßt. Das auf diese Weise gereinigte, nutschenfeuchte 7-Methoxy-1-aminonaphthalin-hydrogensulfat wird vorerst 48 Stunden an der Luft bei 20 - 25 °C und anschließend noch bei 110 °C im Umlufttrockenschrank getrocknet.
Ausbeute: 90 g (53 % d.Th.)
Zp.: 175 - 180 °C (Dunkelfärbung).

7-Methoxy-1-aminonaphthalin-4-sulfonsäure

(3. Reaktionsstufe)

50 g gereinigtes und getrocknetes 7-Methoxy-1-aminonaphthalin-hydrogensulfat werden im Vakuum (20 mm Hg) in einem rotierenden Reaktionsgefäß 6 Stunden auf 160 - 200 °C erhitzt (Back-Verfahren). Nach dem Abkühlen wird die dunkelbraume, körnige Reaktionsmasse in 1600 ml 0,5 %iger Natronlauge bei 80 - 90 °C aufgenommen. Die braune Lösung, die wenig dunkle, flockige Bestandteile enthält, wird mit 25 g Aktivkohle behandelt, filtriert und auf 50 - 60 °C abgekühlt. Unter Rühren scheidet sich auf Zusatz von 150 ml Eisessig die 7-Methoxy-1-aminonaphthalin-4-sulfonsäure in perlmittglänzende Blättchen ab. Nach dem Abkühlen der

Suspension auf 20 - 25 °C wird der kristalline Niederschlag abgesaugt und an der Luft bei 20 - 25 °C getrocknet.
Ausbeute: 27 g (57,8 % d.Th.)
Fp.: > 250 °C (Verkohlung)

7-Methoxy-2-nitroso-1-naphthol-4-sulfonsäure (Na-Salz)

(4. Reaktionsstufe)

In Natronlauge, hergestellt aus 1575 ml Wasser und 34,5 g Natriumhydroxid, werden unter Rühren 218 g 7-Methoxy-1-aminonaphthalin-4-sulfonsäure bei 20 - 25 °C gelöst und in die entstandene braune Lösung 300 g Natriumdisulfit ($Na_2S_2O_5$)eingetragen, wobei das Natriumsalz der 7-Methoxy-1-aminonaphthalin-4-sulfonsäure in farblosen Kristallen ausfällt und beim Erwärmen der Suspension in Lösung geht. Man erhitzt 20 Stunden lang zum Sieden (102 - 105 °C), läßt anschließend auf ca. 30 °C abkühlen und versetzt die braune Lösung mit 10 %iger Natronlauge bis zur phenolphthalinalkalischen Reaktion. Anschließend wird so lange zum Sieden erhitzt (ca. 3 - 4 Stunden), bis in der Dampfphase kein $NH_3$-Gas mehr nachweisbar ist ("$NH_3$-Verkochung").

Die grünstichig braune alkalische Lösung wird auf 20 - 25 °C abgekühlt und mit ca. 1000 ml 37 %iger Salzsäure kongosauer eingestellt. Unter Temperaturanstieg und Ausscheidung einer geringen Menge einer feindispersen Ausfällung setzt eine starke $SO_2$-Entwicklung ein, die durch 2-stündiges Erhitzen des Reaktionsgemisches auf Siedetemperatur zu Ende geführt wird ("$SO_2$-Verkochung"). Die nach "$NH_3$-und $SO_2$-Verkochung" entstandene 7-Methoxy-1-naphthol-4-sulfonsäure wird nicht in Substanz isoliert. Der Austausch einer $NH_2$-Gruppe durch eine OH-Gruppe bei aromatischen Verbindungen, besonders solchen aus der Naphthalinreihe, über Bisulfit-Additionsverbindungen, ist aus der Literatur als "Bucherer-Reaktion" bekannt.

In die nach der "$SO_2$-Verkochung" erhaltene gelbbraune salzsaure Lösung von ca. 5,5 l werden bis zur positiven Nitritreaktion (Kaliumjodid-Stärkepapier-Probe) ca. 150 ml einer 40 %igen Natriumnitritlösung eingetropft, wobei die Innentemperatur der Reaktionslösung 10 °C nicht überschreiten soll. Sind ca. 10 - 15 % der für die Nitrosierungsreaktion erforderlichen Menge der Natriumnitritlösung zugesetzt, beginnt aus der dunkelroten Lösung die Nitrosoverbindung in mikrokristalliner Form auszufallen. Es wird noch 2 - 3 Stunden bei 5 - 10 °C nachgerührt, dann ohne zu rühren noch 30 - 45 Minuten stehengelassen und dann die Suspension über eine Filternutsche abgesaugt. Die rote Nitrosoverbindung wird auf der Nutsche gut abgepreßt und dann an der Luft bei 20 - 25 °C getrocknet.

Die 7-Methoxy-2-nitroso-1-naphthol-4-sulfonsäure fällt als Natriumsulfonat im Gemisch mit Kochsalz in 92 %iger Qualität an.
Ausbeute: 253 g (86 % d.Th.) - bezogen auf 7-Methoxy-1-aminonaphthalin-4-sulfonsäure

7-Methoxy-2-amino-1-naphthol-4-sulfonsäure

(5. Reaktionsstufe)

In verdünnte Natronlauge, hergestellt aus 8500 ml Wasser und 28 g Natriumhydroxid, werden unter Rühren nacheinander 120 g 7-Methoxy-2-nitroso-1-naphthol-4-sulfonsäure und 18,5 g Natriumborhydrid eingetragen. Die entstandene tiefrote Lösung wird auf 2 -3 °C abgekühlt, der Luftraum über der Lösung durch Stickstoff ersetzt und in die Lösung 250 ml 37 %ige Salzsäure unter kräftigem Rühren einfließen lassen. Unter starker Schaumentwicklung steigt die Innentemperatur der Lösung auf 10 - 15 °C an. Beim Unterschreiten des Neutralpunktes fällt ein mikrokristalliner, farbloser Niederschlag aus, wobei die Farbe der Lösung nach hellgelb umschlägt. Die entstandene Suspension wird noch ca. 15 Minuten bei 10 - 15 °C gerührt und dann unter Stickstoff über eine Filternutsche abgesaugt. Der Nutscheninhalt wird mit Eiswasser gewaschen, gut abgepreßt und im Vakuumexsiccator bei 20 - 25 °C über Phosphorpentoxid getrocknet. Die auf diese Weise hergestellte 7-Methoxy-2-amino-1-naphthol-4-sulfonsäure ist praktisch farblos und verfärbt sich an Luft leicht violett.
Ausbeute: 83,5 g (78 % d.Th.)

7-Methoxy-1,2-naphthochinon-(2)-diazid-4-sulfonsäure

(6. Reaktionsstufe)

39 g pulverisierte 7-Methoxy-2-amino-1-naphthol-4-sulfonsäure werden in 650 ml Wasser suspendiert (pH: 2,5 - 2,6). In diese Suspension läßt man bei 15 - 20 °C eine Lösung aus 0,8 g $CuSO_4$ x 5 $H_2O$ und 62,5 ml 2n-$NaNO_2$-Lösung schnell einfließen (pH: 4,0), wobei die Innentemperatur des Diazotierungsgemisches auf

25 - 28 °C ansteigt (pH: 5,0). Die wenig dunkle Bestandteile enthaltende, braune Lösung wird bei dieser Temperatur noch 2 - 3 Stunden nachgerührt und dann mit einigen Tropfen 37 %-iger Salzsäure auf einen pH von 2,6 - 2,8 eingestellt, weitere 15 Minuten nachgerührt, mit 15 g Aktivkohle behandelt und filtriert. Aus dem klaren, rotbraunen Filtrat wird mit 420 ml 37 %iger Salzsäure die 7-Methoxy-1,2-naphthochinon-(2)-diazid-4-sulfonsäure in Form gelber Nadeln ausgefällt. Man rührt noch 3 Stunden bei 5 - 10 °C und saugt dann das Reaktionsgemisch über eine Filternutsche ab. Der Nutscheninhalt wird mit wenig Eiswasser gewaschen und im Vakuumexiccator bei 20 - 25 °C über Phosphorpentoxid getrocknet.
Ausbeute: 24,8 g (61,3 % d.Th.)

7-Methoxy-1,2-naphthochinon-(2)-diazid-4-sulfonsäurechlorid

(7. Reaktionsstufe)

7-Methoxy-1,2-naphthochinon-(2)-diazid-4-sulfonsäure wird entsprechend bekannter Verfahren bei erhöhter Temperatur mit Chlorsulfonsäure zum entsprechenden Sulfonsäurechlorid umgesetzt und anschließend die überschüssige Chlorsulfonsäure mit Eis/Wasser zersetzt. Der anfallende mikrokristalline Niederschlag wird abgesaugt, mit Eiswasser neutral gewaschen und an der Luft bei 20 - 25 °C lichtgeschützt getrocknet. Eine zusätzliche Reinigung des so erhaltenen Sulfonsäurechlorids ist für die Weiterverarbeitung nicht erforderlich.
Ausbeute: 11,4 g (75 % d.Th.)
Zp.: 163 - 165 °C (Dunkelfärbung - Gasentwicklung)

Kondensationsprodukt aus 7-Methoxy-1,2-naphthochinon-(2)-diazid-4-sulfonsäurechlorid und 2,3,4-Trihydroxybenzophenon

(8. Reaktionsstufe)

7-Methoxy-1,2-naphthochinon-(2)-diazid-4-sulfonsäurechlorid wird entsprechend bekannter Verfahren in einem mit Wasser mischbaren organischen Lösemittel, z.B. Aceton, bei 20 - 25 °C mit 2,3,4-Trihydroxybenzophenon in Anwesenheit eines HCl-Akzeptors, z.B. Triethylenamin, N-Methylmorpholin oder Soda, verestert. Die Isolierung des 2,3,4-Trihydroxybenzophenonesters erfolgt in bekannter Weise durch Eintropfen des Versterungsgemisches in 3 %ige Salzsäure. Die hellgelbe amorphe Ausfällung wird abgesaugt, gut abgepreßt und lichtgeschützt an der Luft bei 20 - 25 °C getrocknet. Das auf diese Weise hergestellte Kondensationsprodukt enthält 95 % 2,3,4-Trihydroxybenzophenontrisester und 5 % 2,3,4-Trihydroxybenzophenonbisester der 7-Methoxy-1,2-naphthochinon-(2)-diazid-4-sulfonsäure.
Ausbeute: 20,1 g (88 % d.Th.)

Synthesebeispiel 2

Synthese des Arylesters aus 2,3,4-Trihydroxybenzophenon und 8-Methoxy-1,2-naphthochinon-(2)-diazid-4-sulfonsäure

1-Acetylamino-8-naphthol-4-sulfonsäure

(1. Reaktionsstufe)

Handelsübliche 1-Amino-8-naphthol-4-sulfonsäure (BAYER AG) wird analog 1-Amino-8-naphthol-3,6-disulfonsäure (H-Säure) in wäßriger Sodalösung mit Essigsäureanhydrid bei 50 °C umgesetzt, wobei sowohl die Amino- als auch die Hydroxylgruppe acetyliert werden. Vollständige Acetylierung liegt vor, wenn eine mit Salzsäure angesäuerte Probe des Acetylierungsgemisches bei Zusatz eines Tropfens $NaNO_2$-Lösung und anschließendem Alkalisieren mit Sodalösung keine Blaufärbung ("Selbstkupplung" der diazotierten H-Säure) mehr auftritt, sondern nur noch eine Gelbfärbung zu beobachten ist. Ist dieser Punkt erreicht, wird durch Zusatz von kalzinierter Soda und Erwärmen des Acetylierungsgemisches auf 90 - 95 °C der an Sauerstoff gebundene Acetylrest abgespalten, während die Acetylaminogruppe nicht angegriffen wird. Aus der sodaalkalischen Lösung wird mit 20 %iger Salzsäure die feste, farblose 1-Acetylamino-8-naphthol-4-sulfonsäure ausgefällt, abgesaugt und an der Luft bei 20 - 25 °C getrocknet.
Ausbeute: 90 - 95 % der Theorie

1-Acetylamino-8-methoxynaphthalin-4-sulfonsäure (Na-Salz)

(2. Reaktionsstufe)

1-Acetylamino-8-naphthol-4-sulfonsäure wird nach bekannten Verfahren in 15 %iger Natronlauge mit Dimethylsulfat bei 30 - 40 °C am phenolischen Sauerstoff methyliert, wobei der pH-Wert des Reaktionsgemisches immer größer 8 sein soll. Endpunkt der Methylierungsreaktion: Negative Azofarbstoffkupplung der wäßrigen Phase des Reaktionsgemisches, z.B. mit 4-N,N-Diethylamino-benzoldiazoniumchlorid ($ZnCl_2$-Doppelsalz). Bei unvollständiger Methylierung ist die Azofarbstoffkupplung positiv (Blaufärbung). Während der Methylierungsreaktion fällt die 1-Acetylamino-8-methoxy-naphthalin-4-sulfonsäure als Na-Salz aus dem Reaktionsgemisch in fast farblosen Mikrokristallen aus.
Ausbeute: 70 - 75 % d. Theorie

8-Methoxy-2-aminonaphthalin-4-sulfonsäure

(3. Reaktionsstufe)

8-Methoxy-1-acetylaminonaphthalin-4-sulfonsäure wird in 20 %iger Salzsäure suspendiert und die Suspension am Rückflußkühler unter Rühren auf 90 - 100 °C erhitzt. Nach ca. 1 - 1 1/2 Stunden ist die Entacetylierung vollständig. Man läßt auf 20 - 25 °C abkühlen, saugt die schwach rosa gefärbten Kristalle ab, wäscht diese auf der Nutsche mit Wasser und trocknet sie dann im Vakuumtrockenschrank bei 50 - 60 °C.
Ausbeute: 90 - 95 % d.Theorie

8-Methoxy-1-naphthol-4-sulfonsäure

(4. Reaktionsstufe)

Der Austausch der Aminogruppe gegen die phenolische Hydroxylgruppe gelingt analog der aus der Literatur bekannten Bucherer-Reaktion. 8-Methoxy-1-aminonaphthalin-4-sulfonsäure wird in handelsüblicher 37 %iger wäßriger $NaHSO_3$-Lösung bei 100 °C ca. 20 - 24 Stunden unter Rühren am Rückflußkühler erwärmt. Die enstandene Suspension wird anschließend mit 10 %iger Natronlauge phenolphthaleinalkalisch gestellt und unter weiterem Rühren solange zum Sieden erhitzt (ca. 3 - 4 Stunden), bis in der Dampfphase des Reaktionsgefäßes kein $NH_3$-Gas mehr nachweisbar ist ("$NH_3$-Verkochung). Man kühlt die Suspension anschließend auf 20 - 25 °C ab und stellt diese mit 37 %iger Salzsäure kongosauer. Unter Temperaturanstieg setzt sofort eine starke $SO_2$-Entwicklung ein. Nach 2-stündigem Erhitzen des Reaktionsgemisches auf Siedetemperatur ist die $SO_2$-Entwicklung beendet ("$SO_2$-Verkochung"). Das Reaktionsgemisch wird auf 20 - 25 °C abgekühlt und die Suspension abgesaugt. Die auf der Filternutsche gesammelten, fast farblosen Kristalle werden mit wenig kaltem Wasser gewaschen und im Vakuumtrockenschrank bei 70 - 75 °C getrocknet.
Ausbeute: 75 - 80 % d.Theorie

8-Methoxy-2-nitroso-1-naphthol-4-sulfonsäure (Na-Salz)

(5. Reaktionsstufe)

8-Methoxy-1-naphthol-4-sulfonsäure wird in 37 %iger Salzsäure/Wasser (1:4) suspendiert und unter Rühren mit einer 2n-$NaNO_2$-Lösung bei 0 - 5 °C bis zur positiven Nitritreaktion (Kaliumjodid-Stärkepapier-Test) nitrosiert. Nach ca. 2 - 2 1/2 Stunden ist die Nitrosierung vollständig. Die entstandene gelbe mikrokristalline Nitrosoverbindung wird abgesaugt und die noch nutschenfeuchte Substanz durch Lösen in Wasser und Ausfällen mit Kochsalz gereinigt. Die so erhaltene Nitrosoverbindung wird im Vakuumtrockenschrank bei 30 - 35 °C getrocknet.
Ausbeute: 80 - 85 % d.Theorie

8-Methoxy-2-amino-1-naphthol-4-sulfonsäure

(6. Reaktionsstufe)

8-Methoxy-2-nitroso-1-naphthol-sulfonsäure wird in 37 %-iger wäßriger $NaHSO_3$-Lösung suspendiert und unter Rühren am Rückflußkühler ca. 1 Stunde auf 80 - 85 ° C erwärmt. Die hierbei entstandene hellbraune

Lösung wird auf 20 - 25 °C abgekühlt und mit 37 %iger Salzsäure/Wasser (2:1) kongosauer eingestellt. Die spontan einsetzende $SO_2$-Entwicklung wird durch Erwärmen der Reaktionslösung auf 80 - 85 °C zu Ende geführt, wobei gleichzeitig die 8-Methoxy-1-amino-1-naphthol-4-sulfonsäure als schwer lösliches inneres Salz (Betain) ausfällt. Nach Abkühlen des Reaktionsgemisches auf 20 - 25 °C wird der schwach rosa gefärbte Niederschlag abgesaugt, mit Wasser neutral gewaschen und im Vakuumtrockenschrank bei 40 - 45 °C getrocknet.
Ausbeute: 75 - 80 % d.Theorie

8-Methoxy-1,2-naphthochinon-(2)-diazid-4-sulfonsäure

(7. Reaktionsstufe)

8-Methoxy-2-amino-1-naphthol-4-sulfonsäure wird in Wasser suspendiert, die Suspension mit 10 %iger $NaHCO_3$-Lösung auf eine pH-Wert von 4,0 - 4,5 eingestellt und anschließend in die Suspension eine wäßrige Lösung aus $NaNO_2$ und $CuSO_4$ rasch eingetropft, wobei die Temperatur des Diazotierungsgemisches auf 15 - 20 °C begrenzt wird. Die Diazotierung ist vollständig, sobald eine schwache, bleibende Nitritreaktion (Kaliumjodid-Stärkepapier-Test) festzustellen ist. Das für die Diazotierung erforderliche $CuSO_4 \cdot 5H_2O$ wird in einer Menge von 1,0 - 1,5 %, bezogen auf die eingesetzte Aminonaphthol-4-sulfonsäure, verwendet. Die Diazolösung wird noch 30 Minuten bei 15 - 20 °C gerührt, der pH-Wert auf 5 - 6 eingestellt und über Aktivkohle filtriert. Aus dem klaren, gelbbraunen Filtrat wird durch Zusatz von Kochsalz die gelbgefärbte, kristalline 8-Methoxy-1,2-naphthochinon-(2)-diazid-4-sulfonsäure als Na-Salz ausgefällt. Man läßt noch 30 Minuten bei 20 - 25 °C ohne Rühren stehen und saugt dann das Naphthochinondiazid ab, wäscht dieses auf der Nutsche mit wenig Eiswasser und trocknet dann den Nutscheninhalt im Vakuumtrockenschrank bei 30 - 35 °C.
Ausbeute: 70 - 75 % d.Theorie

8-Methoxy-1,2-naphthochinon-(2)-diazid-4-sulfonsäurechlorid

(8. Reaktionsstufe)

8-Methoxy-1,2-naphthochinon-(2)-diazid-4-sulfonsäure (Na-Salz) wird analog des im Synthese-Beispiels 1 beschriebenen Chlorierungsverfahrens mit Chlorsulfonsäure zum entsprechenden Sulfonsäurechlorid umgesetzt, anschließend die im Überschuß verwendete Chlorsulfonsäure mit Eis/Wasser zersetzt und der anfallende, gelbgefärbte, mikrokristalline Niederschlag abgesaugt. Dieser wird auf der Filternutsche mit Eiswasser neutral gewaschen und anschließend im Vakuumtrockenschrank bei 20 - 25 °C getrocknet. Für die Weiterverarbeitung des Sulfonsäurechlorids ist eine Reinigung nicht erforderlich.
Ausbeute: 85 -90 % d.Theorie
Zp.: 163 - 165 °C (Dunkelfärbung, Gasentwicklung)

Kondensationsprodukt aus 8-Methoxy-1,2-naphthochinon-(2)-diazid-4-sulfonsäurechlorid und 2,3,4-Trihydroxy-benzophenon

(9. Reaktionsstufe)

Die Kondensation erfolgt analog des im Synthesebeispiels 1 beschriebenen Verfahrens in Aceton bei 20 - 25 °C in Anwesenheit eines HCl-Akzeptors. Die Isolierung des veresterten 2,3,4-Trihydroxybenzophenons gelingt durch Eintropfen des Gemisches in 3%ige Salzsäure. Die hellgelbe, amorphe Ausfällung wird abgesaugt, gut abgepreßt, mit Wasser gewaschen und im Vakuumtrockenschrank bei 20 - 25 °C getrocknet.
Ausbeute: 85 -90 % d.Theorie
(93 % Trisester, 7 % Diester)
Zp.: 170 °C (Dunkelfärbung)

Anwendungsbeispiel 1

Mit einer Lösung von

| | |
|---|---|
| 72,10 Gt | Propylenglykolmethyletheracetat |
| 2,24 Gt | Verbindung Nr. 1 (Tabelle 2) |
| 1,24 Gt | 2,6-Bis-hydroxymethyl-p-kresol |
| 24,41 Gt | Kresol-Formaldehyd-Novolak (Schmelzbereich 122 - 132 °C nach DIN 53 181) |

werden Siliciumwafer auf einer Lackschleuder bei einer Drehzahl von 4000 U/min beschichtet und anschließend auf einer Heizplatte bei einer Temperatur von 110 °C 60 s lang getrocknet. Die Schichtdicke beträgt ca. 1,5 µm. Mit einem Projektionsbelichtungsgerät vom Typ FPA 1550 der Firma Canon wird bei einer Wellenlänge von 436 nm durch eine Photomaske, die verschiedene Strichgitter im Größenbereich von 2,0 bis 0,65 µm enthält, belichtet. Die belichteten Wafer werden in einem Tauchverfahren mit einem Entwickler der Marke "AZ-Developer 30" (Hersteller HOECHST AG) 60 s lang entwickelt, anschließend mit Wasser gespült und getrocknet. Man erhält ein positives Abbild der Photomaske, wobei bei einer Belichtungsenergie von 120 $mJ/cm^2$ noch Linien und Gräben der Breite 0,9 µm einwandfrei aufgelöst sind.

Wird in der obengenannten Formulierung 2,6-Bishydroxymethyl-p-kresol durch gleiche Gewichtsmengen Kresol-Formaldehyd-Novolak ersetzt, wird das lithographische Ergebnis nur unwesentlich beeinflußt.

Anwendungsbeispiel 2

Anwendungsbeispiel 1 wird wiederholt, wobei die Siliciumwafer nach der Belichtung 60 s lang bei einer Temperatur von 120 °C auf einer Heizplatte getempert und danach einer ganzflächigen Belichtung von 200 $mJ/cm^2$ mittels eines Kontaktbelichters des Typs Sues MJB 3 ausgesetzt werden. Die Messung dieser Belichtungsenergie erfolgt mit einem Intensitätsmeßgerät der Firma OAI, Modell 206, für den Wellenlängenbereich von 400 bis 500 nm. Nach der Entwicklung erhält man ein negatives Abbild der Photomaske, wobei bei einer Belichtungsenergie von 120 $mJ/cm^2$ noch Linien und Gräben der Breite 0,6 µm einwandfrei aufgelöst sind.

Anwendungsbeispiel 3

Anwendungsbeispiel 1 wird wiederholt, wobei die Zusammensetzung der Photoresistlösung wie folgt geändert wird:

| | |
|---|---|
| 72,10 Gt | Propylenglykolmethyletheracetat |
| 3,08 Gt | Verbindung Nr. 1 |
| 1,24 Gt | 2,6-Bis-hydroxymethyl-p-kresol |
| 23,58 Gt | Kresol-Formaldehyd-Novolak |

Man erhält wiederum ein positives Abbild der Photomaske. Bei einer Belichtungsenergie von 180 $mJ/cm^2$ werden Linien und Gräben der Größe 0,9 µm klar aufgelöst.

Anwendungsbeispiel 4

Anwendungsbeispiel 3 wird wiederholt, wobei analog zu Anwendungsbeispiel 2 nach der Belichtung durch die Photomaske wieder ein einminütiger Temperschritt bei 120 °C auf einer Heizplatte und nach dem Abkühlen eine ganzflächige Belichtung von 200 $mJ/cm^2$ durchgeführt wird. Im nach der Entwicklung sichtbaren negativen Abbild der Photomaske sind Linien und Gräben bis herab zu 0,65 µm aufgelöst, wenn die Energie der ersten Belichtung bei 180 $mJ/cm^2$ liegt.

Anwendungsbeispiel 5

Die Anwendungsbeispiele 1 bis 4 werden wiederholt, wobei die Verbindung Nr. 1 durch eine gleiche Gewichtsmenge der Verbindung 9 ersetzt wird. Unter einer Anpassung der Belichtungszeit an die speziellen Eigenschaften der einzelnen Lösungen lassen sich sowohl mit der Positiv- als auch der Negativ-Verarbeitung vergleichbar gute Resultate erzielen. Die gleichen guten Resultate werden auch erreicht, wenn man Gemische aus den beiden Verbindungen Nr. 1 und Nr. 9 einsetzt.

Die Zugabe oberflächenaktiver Additive zu diesen Lösungen zur Ernzielung einer, nach der Beschichtung völlig glatten Oberfläche, beeinflußt die lithographischen Eigenschaften nicht.

Zum Vergleich:

Wird anstelle der Verbindungen Nr. 1 und Nr. 9 unsubstituierter 1,2-Naphthochinon(2)-diazid-4-sulfonsäureester (Verbindung Nr. 0) eingesetzt, so liegen die erforderlichen Belichtungsenergien bei Verwendung von Licht der Wellenlänge 436 nm um den Faktor 5 bis 14 höher. Die folgende Tabelle 3 gibt die erforderlichen Belichtungsenergien sowohl für die Positiv- als auch für die Negativ-Verarbeitung wieder:

Tabelle 3:

| Verbindung Nr. | Gehalt in der Lösung (%) | Verarbeitung | Belichtungenergie ($mJ/cm^2$) |
|---|---|---|---|
| 1 | 2,24 | pos. | 120 |
| 1 | 2,24 | neg. | 120 |
| 1 | 3,08 | pos. | 180 |
| 1 | 3,08 | neg. | 180 |
| 9 | 2,24 | pos. | 180 |
| 9 | 2,24 | neg. | 180 |
| 9 | 3,08 | pos. | 220 |
| 9 | 3,08 | neg. | 220 |
| 0 | 2,24 | pos. | 1600 |
| 0 | 2,24 | neg. | 1100 |
| 0 | 3,08 | pos. | 1700 |
| 0 | 3,08 | neg. | 1400 |

Anwendungsbeispiel 6

Mit einer Lösung aus

| | |
|---|---|
| 72,10 Gt | Propylenglykolmethyletheracetat |
| 2,77 Gt | Verbindung Nr. 11 |
| 1,10 Gt | 2,6-Bis-hydroxymethyl-p-kresol |
| 24,00 Gt | Kresol-Formaldehyd-Novolak |

werden Siliciumwafer wie in Anwendungsbeispiel 1 beschichtet, getrocknet und anschließend belichtet. Nach einer Entwicklung innerhalb von einer Minute ergibt sich ein positives Abbild der Photomaske, wobei Linien und Gräben bis zu 0,8 µm einwandfrei aufgelöst sind. Wird nach der Belichtung ein Temperschritt von 60 s Dauer bei einer Temperatur von 125 °C auf einer Heizplatte und eine ganzflächige Belichtung von 200 $mJ/cm^2$ durchgeführt, so ist beim folgenden Entwicklungvorgang nach ca. 45 s ein negatives Abbild der Maske zu sehen. Die Resistenz dieses Bildes gegen den Angriff des Entwicklers ist jedoch nur gering, so daß bei Verlängerung der Entwicklungszeit die Schicht vollständig von den Siliciumwafern abgelöst wird. Mit angepaßten Belichtungsenergien und Entwicklungszeiten zeigt sich dieses Verhalten auch, wenn die Verbindung Nr. 11 durch die Verbindung Nr. 6 oder Nr. 13 ersetzt wird.

Anwendungsbeispiel 7

Mit einer Lösung aus

| | |
|---|---|
| 72,10 Gt | Propylenglykolmethyletheracetat |
| 3,62 Gt | Verbindung Nr. 3 |
| 1,14 Gt | 2,6-Bis-hydroxymethyl-p-kresol |
| 23,14 Gt | Kresol-Formaldehyd-Novolak |

werden Siliciumwafer wie in Anwendungsbeispiel 1 beschichtet, getrocknet, belichtet. Anschließend werden sie einer Temperaturbehandlung von einer Minute Dauer bei 120 °C auf einer Heizplatte und nach dem Abkühlen einer ganzflächigen Belichtung von 200 $mJ/cm^2$ ausgesetzt. Nach einer Entwicklung von 45 s Dauer ergibt sich ein negatives Abbild der Belichtungsvorlage mit einer Auflösung besser als 0,9 µm, wenn die Energie der bildmäßigen Belichtung 400 $mJ/cm^2$ beträgt. Das gleiche Ergebnis wird auch erzielt, wenn die Zusammensetzung der Photoresistlösung wie folgt geändert wird:

72,36 Gt Propylenglykolmethyletheracetat
2,16 Gt Verbindung Nr. 4
1,08 Gt 2,6-Bis-hydroxymethyl-p-kresol
24,40 Gt Kresol-Formaldehyd-Novolak

Anwendungsbeispiel 8

Siliciumwafer werden mit eine Lösung aus

72,11 Gt Propylenglykolmethyletheracetat
2,77 Gt Verbindung Nr. 5
1,27 Gt 2,6-Bis-hydroxymethyl-p-kresol
23,85 Gt Kresol-Formaldehyd-Novolak

wie in Anwendungsbeispiel 1 beschichtet, getrocknet belichtet. Eine Entwicklung von 4 Minuten löst in dem positiven Maskenabbild Linien und Gräben bis herab zu 0,8 µm auf, wenn die Belichtungsenergie 280 mJ/cm² beträgt. Führt man vor der Entwicklung eine Temperaturbehandlung von einer Minute bei 120 °C auf einer Heizplatte und anschließend eine ganzflächige Belichtung von 20 mJ/cm² durch, so erhält man nach einer Entwicklung von einer Minute ein negatives Maskenbild. Die Auflösung ist besser als 0,7 µm, wenn die Belichtungsenergie bei 360 mJ/cm² liegt. Das gleiche Verhalten zeigt sich auch, wenn eine Lösung folgender Zusammensetzung verwendet wird:

72,10 Gt Propylenglykolmethyletheracetat
2,51 Gt Verbindung Nr. 2
1,10 Gt 2,6-Bis-hydroxymethyl-p-kresol
24,29 Gt Kresol-Formaldehyd-Novolak

Bei der Negativ-Verarbeitung liegt die erforderliche Belichtungsenergie bei 200 mJ/cm².

Anwendungsbeispiel 9

Analog zu Anwendungsbeispiel 1 werden Siliciumwafer mit einer Lösung aus

72,03 Gt Propylenglykolmethyletheracetat
2,24 Gt Verbindung Nr. 1
1,24 Gt 2,6-Bis-hydroxymethyl-p-kresol
24,39 Gt Kresol-Formaldehyd-Novolak
0,10 Gt 4-(Di-2-acetoxyethyl)-amino-2-methyl-α-cyanozimt-säurenitril

beschichtet, getrocknet und belichtet. Nach einer Entwicklungszeit von einer Minute sind im entstandenen positiven Maskenbild Strukturen bis 0,9 µm aufgelöst.

Wird nach einer Belichtung noch eine Temperaturbehandlung von 60 s bei 120 °C auf einer Heizplatte und eine ganzflächige Belichtung von 200 mJ/cm² durchgeführt, so entsteht unter den gleichen Bedingungen ein negatives Abbild der Photomaske, das noch gut aufgelöste 0,6 µm, Strukturen zeigt.

Anwendungsbeispiel 10

Siliciumwafer werden mit der Photoresistlösung des Anwendungsbeispiels 1 beschichtet und anschließend 1 Minute bei 80 °C auf einer Heizplatte getrocknet und wie beschrieben belichtet. Zur Entwicklung wird eine 0,27 n wäßrige Lösung von Tetramethyl-ammonium-hydroxid benutzt. Nach einer Entwicklungszeit von 30 s sind im positiven Abbild der Photomaske Linien und Gräben bis zu 0,9 µm aufgelöst. Wird nach der Entwicklung ein Temperprozeß von 60 s bei 120 °C auf einer Heizplatte und eine ganzflächige Belichtung von 200 mJ/cm² durchgeführt, so sind nach 45 s Entwicklungszeit im entstandenen negativen Abbild der Maske Linien und Gräben bis zu 0,75 µm aufgelöst.

Anwendungsbeispiel 11

Siliciumwafer werden mit der Photoresistlösung des Anwendungsbeispiels 1 beschichtet, eine Minute bei 100 °C getrocknet und wie beschrieben belichtet. An eine einminütige Temperaturbehandlung bei 125 °C schließt sich unmittelbar, also ohne ganzflächige Nachbelichtung, eine Entwicklung in 0,4 n wäßriger Tetramethylammoniumhydroxidlösung an. Nach 45 s Entwicklungszeit liegt ein negatives Abbild der Maske vor, wobei die Auflösung bei 120 mJ/cm² Belichtungsenergie besser als 0,9 µm ist.

Anwendungsbeispiel 12

Die Zusammensetzung der Photoresistlösung des Anwendungsbeispiels 1 wird abgeändert, indem das 2,6-Bis-hydroxymethyl-p-kresol durch eine gleiche Gewichtsmenge an Hexaalkoxymethylmelamin (Alkoxy = $CH_3O$- / $C_4H_9O$- 1:1) ersetzt wird. Mit dieser Lösung werden Siliciumwafer wie in Anwendungsbeispiel 1 beschichtet, getrocknet und belichtet. Nach einer Entwicklungszeit von 2 Minuten liegt ein positives Maskenbild mit einer Auflösung von 0,9 µm vor, wenn die Belichtungsenergie 360 mJ/cm² beträgt.

Wird nach der Belichtung eine Temperaturbehandlung von 60 s Dauer bei 120 °C und eine ganzflächige Belichtung von 200 mJ/cm² durchgeführt, so entsteht bei einer vierminütigen Entwicklung ein negatives Maskenabbild. Die Auflösung von 0,7 µm wird erreicht, wenn zuvor 200 mJ/cm² belichtet wurde.

Anwendungsbeispiel 13

Siliciumwafer werden mit einer Lösung aus

| | |
|---|---|
| 72,10 Gt | Cyclohexanon |
| 2,51 Gt | Verbindung Nr. 16 |
| 1,34 Gt | 2,6-Bis-hydroxymethyl-p-kresol |
| 24,05 Gt | Kresol-Formaldehyd-Novolak |

wie in Anwendungsbeispiel 1 beschichtet, getrocknet und belichtet. Eine Entwicklung von einer Minute löst in dem positiven Maskenabbild Linien und Gräben bis herab zu 0,8 µm auf, wenn die Belichtungsenergie 59 mJ/cm² beträgt. Führt man vor der Entwicklung eine Temperaturbehandlung von einer Minute bei 120 °C auf einer Heizplatte und anschließend eine ganzflächige Belichtung von 200 mJ/cm² durch, so erhält man nach einer Entwicklung von einer Minute ein negatives Maskenabbild. Die Auflösung ist besser als 0,7 µm, wenn die Belichtungsenergie bei 70 mJ/cm² liegt.

Anwendungsbeispiel 14

Mit einer Lösung aus

| | |
|---|---|
| 72,10 Gt | Propylenglykolmethyletheracetat |
| 2,23 Gt | Verbindung Nr. 1 |
| 2,79 Gt | 2,6-Bis-hydroxymethyl-n-propyl-phenol |
| 22,88 Gt | Kresol-Formaldehyd-Novolak |

werden Siliciumwafer wie in Anwendungsbeispiel 1 beschichtet, getrocknet und belichtet. Nach der Belichtung wird eine Temperaturbehandlung von 60 s Dauer bei 120 °C und anschließend eine ganzflächige Belichtung von 200 mJ/cm² durchgeführt. Nach einer Entwicklung von einer Minute Dauer liegt ein negatives Abbild der Maske vor. Die Auflösung ist besser als 0,65 µm, wenn die Strukturbelichtungsenergie 64 mJ/cm² beträgt. Das gleiche Ergebnis wird erzielt, wenn das 2,6-Bishydroxymethyl-n-propyl-phenol durch eine gleiche Gewichtsmenge von 2,6-Bishydroxymethyl-4-(2-phenyl-2-propyl)-phenol ersetzt wird. Die erforderliche Belichtungsenergie liegt dann bei 300 mJ/cm².

Wird das 2,6-Bishydroxymethyl-n-propyl-phenol durch eine Menge von 4,19 Gt 2,6-Bishydroxymethyl-4-ethyl-phenol ersetzt und gleichzeitig die Menge des Kresol-Formaldehyd-Novolaks von 22,88 Gt auf 21,48 Gt erniedrigt, so ergibt sich unter den gleichen Versuchsbedingungen eine Auflösung von 0,9 µm, wobei die erforderliche Belichtungsenergie bei nur 48 mJ/cm² liegt.

Anwendungsbeispiel 15

A. Molare Extinktionskoeffizienten (ε) einiger 1,2-Naphthochinon-(2)-diazid-4-sulfonsäureesterderivate gemäß der Erfindung

Tabelle 4 (Numerierung entsprechend Tabelle 2)

| Nr. der Verbindung | Molgewicht MG | $\epsilon$ $l \cdot cm^{-1} \cdot mol^{-1}$ bei $\lambda$ = 436 nm | $\epsilon$ $l \cdot cm^{-1} \cdot mol^{-1}$ bei $\lambda_{max}$ |
|---|---|---|---|
| 0 (Vgl.) | 926 | 400 | 10 700 (375 nm) |
| 1 | 1016 | 4 700 | 16 800 (395 nm) |
| 2 | 1016 | 2 370 | 29 250 (388 nm) |
| 3 | 1016 | 420 | 17 500 (368 nm) |
| 4 | 968 | 700 | 16 650 (375 nm) |
| 5 | 1010 | 900 | 20 100 (383 nm) |
| 6 | 474 | 1 200 | 6 800 (392 nm) |
| 9 | 1038 | 4 450 | 17 400 (394 nm) |
| 11 | 882 | 2 000 | 13 000 (392 nm) |
| 13 | 836 | 3 400 | 12 300 (395 nm) |
| 16 | 1097 | 5 880 | 17 750 (396 nm) |
| 18 | 1100 | 1 300 | 19 030 (385 nm) |
| 14 (Vgl.) | 1033 | 390 | 16 900 (375 nm) |

B. Normierte" molare Extinktionskoeffizienten (ε') der in Tabelle 4 aufgeführen Verbindungen.

Tabelle 5 (Numerierung entsprechend Tabelle 4)

| Nr. der Verbindung | Molgewicht $M_D$* | ε' [l · $cm^{-1}$ · $mol^{-1}$] λ = 436 nm | ε' [l · $cm^{-1}$ · $mol^{-1}$] $\lambda_{max}$ |
|---|---|---|---|
| 0 (Vgl.) | 233 | 101 | 2692 |
| 1 | 263 | 1217 | 4349 |
| 2 | 263 | 614 | 7572 |
| 3 | 263 | 109 | 4530 |
| 4 | 247 | 179 | 4249 |
| 5 | 261 | 233 | 5194 |
| 6 | 263 | 666 | 3773 |
| 9 | 263 | 1128 | 4409 |
| 11 | 263 | 596 | 3876 |
| 13 | 263 | 1070 | 3870 |
| 14 (Vgl.) | 269 | 102 | 4401 |
| 16 | 290 | 1554 | 4692 |
| 19 | 291 | 344 | 5034 |

Unter der "normierten" molaren Extinktion ε' soll der Anteil der molaren Extinktion ε des 1,2-Naphthochinon-(2)-diazid-4-sulfonsäure-Derivats $M_D$ verstanden werden, der auf einen 1,2-Naphthochinon-(2)-diazid-4-sulfonsäurerest D* entfällt,

D* =
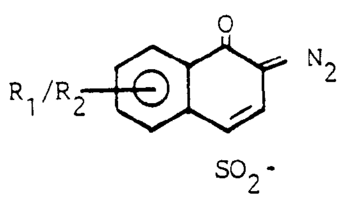

wobei $R_1$ und $R_2$ die oben angegebene Bedeutung haben und vorausgesetzt wird, daß der Betrag der Eigenabsorption des Ester- oder Amidrestes $M_{E/A}$ bei der infrage kommenden Wellenlänge vernachlässigbar ist. Das Molgewicht $M_{D*}$ des 1,2-Naphthochinon-(2)-diazid-4-sulfonsäurerestes D* berechnet sich nach

$$M_{D*} = \frac{M_D - M_{E/A}}{n}$$

wobei

$M_D$ = Molgewicht des 1,2-Naphthochinon-(2)-diazid-4-sulfonsäure-Derivats
$M_{E/A}$ = Molgewicht des Ester- bzw. Amidrestes und
n = Anzahl der 1,2-Naphthochinon-(2)-diazid-4-sulfonsäure-Einheiten.

bedeuten.

Die "normierte" Extinktion ε' berechnet sich nach

$$\varepsilon' = \frac{M_{D*}}{M_D} \cdot \varepsilon$$

wobei

ε = molare Extinktion des 1,2-Naphthochinon-(2)-diazid-4-sulfonsäure-Derivats

bedeutet.

Die Werte der in der Tabelle 5 aufgeführten "normierten" molaren Extinktionskoeffizienten ε' übersteigen die molaren Extinktionskoeffizienten ε der Vergleichsverbindungen (Nr. 0 und Nr. 14) z.T. beträchtlich und erfüllen damit die für das Eintreten einer Lichtreaktion notwendige Voraussetzung hinreichend hoher Absorption in stärkerem Maße als es für die Vergleichsbedingungen der Fall ist. Alle Verbindungen im Sinne der Erfindung deren normierter molarer Extinktionskoeffizient den Wert ε' = 120 übersteigt, sind besonders bevorzugt.

Anwendungsbeispiel 16

Siliciumwafer werden mit einer Lösung aus

72,11 Gt Propylenglykolmethyletheracetat
7,81 Gt Verbindung Nr. 1
20,09 Gt Kresol-Formaldehyd-Novolak

wie in Anwendungsbeispiel 1 beschichtet und bei einer Temperatur von 90 °C 45 s lang auf einer Heizplatte getrocknet. Die Belichtung erfolgt mit einem Projektionsbelichter der Firma ASM mit einer numerischen Apertur von 0.42 bei einer Wellenlänge von 365 nm. Nach einer zweiten Temperaturbehandlung von 45 s Dauer bei einer Temperatur von 100 °C auf einer Heizplatte wird 30 s lang mit einem Entwickler der Marke "AZ 524 MIF" (Hersteller Hoechst AG) im Tauchervfahren entwickelt, mit Wasser gespült und getrocknet. Man erhält ein positives Abbild der Photomaske, wobei bei einer Belichtungsenergie von 95 mJ/cm² die kleinsten auf der Maske vorhandenen Strukturen der Breite 0.7 µm, einwandfrei aufgelöst sind.

Anwendungsbeispiel 17

Siliciumwafer werden mit einer Lösung aus

72,11 Gt Propylenglykolmethyletheracetat
2,79 Gt Verbindung Nr. 1
1,67 Gt 2,6-Bis-hydroxymethyl-p-ethyl-phenol
23,44 Gt Kresol-Formaldehyd-Novolak

wie in Anwendungsbeispiel 1 beschichtet und getrocknet. Die Belichtung erfolgt mit einem Projektionsbelichter der Firma ASM mit einer numerischen Apertur von 0.42 bei einer Wellenlänge von 365 nm. Anschließend wird eine Temperaturbehandlung von 60 s Dauer auf einer Heizplatte bei 120 °C und nach dem Abkühlen eine ganzflächige Belichtung von 200 mJ/m² durchgeführt. Die Entwicklung erfolgt während 80 s in einem Entwickler der Marke "AZ 524 MIF" (Hersteller Hoechst AG) im Tauchverfahren. Man erhält ein negatives Abbild der Photomaske, wobei bei einer Belichtungsenergie von 350 mJ/cm² Linien und Gräben der Breite 0.5 µm, klar mit steilen Kanten aufgelöst sind.

## Patentansprüche

1. Strahlungsempfindliche Verbindungen, die Amide oder Ester einer 1,2-Naphthochinon-(2)-diazid-4-sulfonsäure der allgemeinen Formel

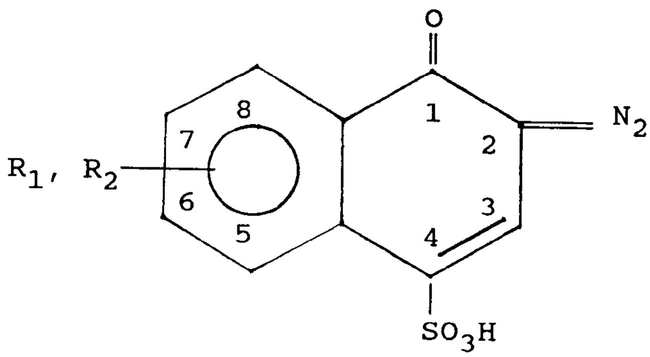

(I)

darstellen, worin

$R_1$ und $R_2$ gleich oder verschieden sind und im Falle von

**Amiden** Wasserstoffatome, Alkyl-, Alkylether- oder Alkylthioethergruppen, deren Kohlenstoffketten durch Ethersauerstoffatome unterbrochen sein können, Acylamino-, Carbonsäureester- oder Sulfonsäureestergruppen,

**Estern** zusätzlich auch Sulfonamidgruppen

bedeuten, mit der Maßgabe, daß $R_1$ und $R_2$ nicht gleichzeitig Wasserstoff sind.

**2.** Verbindungen nach Anspruch 1, worin

$R_1$ und $R_2$ gleich oder verschieden sind und Wasserstoff, eine Alkylgruppe mit 1 bis 6 Kohlenstoffatomen, eine Alkylether- oder Alkylthioethergruppe, deren Kohlenstoffketen durch Ethersauerstoffatome unterbrochen sein können, mit insgesamt 1 bis 6 Kohlenstoffatomen oder eine Acylaminogruppe mit 2 bis 6 Kohlenstoffatomen

bedeuten.

**3.** Verbindungen nach Anspruch 1 oder 2, worin

$R_1$ Wasserstoff und

$R_2$ Alkylether- oder Alkylthioethergruppen, deren Kohlenstoffkette durch Ethersauerstoffatome unterbrochen sein kann, mit 1 bis 6 Kohlenstoffatomen

bedeuten.

**4.** Verbindungen nach Anspruch 1 oder 2, worin

$R_1$ Wasserstoff oder Methyl und

$R_2$ Methyl oder eine Alkylethergruppe mit 1 - 4 Kohlenstoffatomen bedeuten.

**5.** Verbindungen nach Ansprüchen 1 bis 4, worin

$R_1$ in Position 6 Wasserstoff oder Methyl und

$R_2$ in Position 7 Methyl oder die Methylethergruppe bedeuten.

**6.** Verbindungen nach Ansprüchen 1 bis 4, worin

$R_1$ in Position 6 Wasserstoff und

$R_2$ in Position 7 die Methylethergruppe bedeuten.

**7.** Strahlungsempfindliche Verbindungen nach Anspruch 1, dadurch gekennzeichnet, daß die strahlungsempfindlichen Verbindungen Ester sind aus der 1,2-Naphthochinon-(2)-diazid-4-sulfonsäure der Formel I und einer Mono- oder Polyhydroxyarylverbindung der Formel II

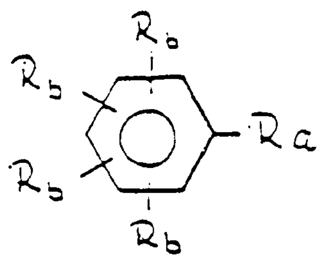

(II)

in der

$R_a$ = H, -X-$R_c$ oder

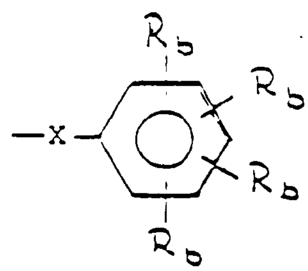

$R_b$ = H, eine OH-Gruppe, Halogen oder eine niedere Alkylgruppe bedeutet, wobei mindestens eine und höchstens sechs $R_b$-Gruppen OH-Gruppen sind,

X = eine -Kohlenstoff-Bindung, - O -, - S -, - $SO_2$-,

$$-\underset{\underset{O}{\|}}{C}-,\ -\underset{\underset{O}{\|}}{C}-(CH_2)_n-,\ -CH_2-\underset{\underset{O}{\|}}{C}-(CH_2)_n,$$

$$-\underset{\underset{O}{\|}}{C}-(CH_2)_n-\underset{\underset{O}{\|}}{C}-,\ -\underset{\underset{O}{\|}}{C}-O-,\ -\underset{\underset{O}{\|}}{C}-O-(CH_2)_n-,$$

$$-CH_2-\underset{\underset{O}{\|}}{C}-O-(CH_2)_n-,\ -(CH_2)_p-,\ CH_3-\underset{|}{\overset{|}{C}}-CH_3,$$

$$CH_3-\underset{|}{\overset{|}{C}}-(CH_2)_n-Y,\ H-\underset{|}{\overset{|}{C}}-(CH_2)_n-Y$$

oder

$$CF_3-\underset{|}{\overset{|}{C}}-CF_3,$$

wobei jeweils einzelne $CH_2$-Gruppen durch Etherfunktionen oder der Wasserstoff durch Substituenten ersetzt sein können, darstellt,

n = 1 oder 2,

P = 1 bis 3,

Y = H oder eine gegebenenfalls substituierte Alkoxy-, Carboxy-, Carboxyalkyl-, Carboxyalkoxyalkyl- oder Arylgruppe und

$R_c$ = H oder eine gegebenenfalls substituierte Alkyl- oder Arylgruppe bedeutet

bedeuten oder

der Formel III

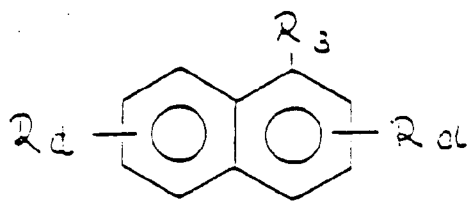

(III)

in der

$R_3$ = H oder

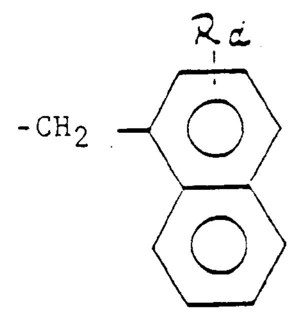

bedeutet und

$R_d$ = H oder eine OH-Gruppe darstellt, wobei mindestens eine der $R_d$-Gruppen eine OH-Gruppe ist oder der Formel IV

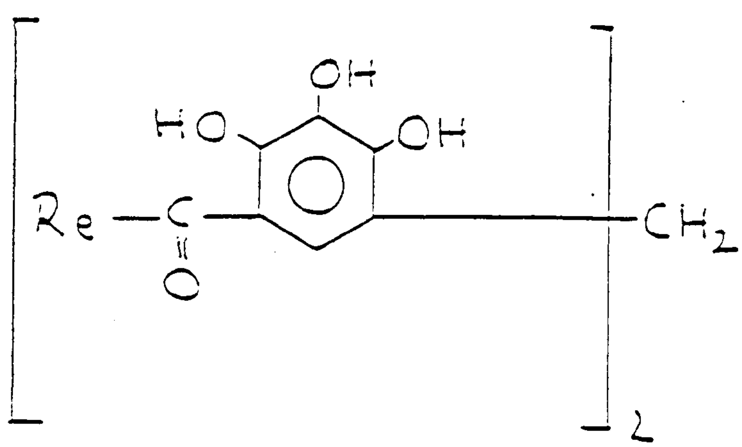

(IV)

in der

$R_e$ = H oder eine gegebenfalls substituierte Alkyl-, Alkoxy- oder Arylgruppe

bedeutet

oder der Formel V

$$R_f\text{ - OH} \qquad \text{(V)}$$

in der

$R_f$ = eine geradkettige oder verzweigte, ggf. durch Halogen oder einen Acylaminorest substituierte Alkyl-, Cycloalkyl- oder Aralkylgruppe mit 1 bis 14 C-Atomen, deren Kohlenstoffkette durch Ethersauerstoffatome unterbrochen sein kann,

oder der Gruppe

- Z - OH

in der

Z = ein Alkylenrest mit 2 bis 12 C-Atomen oder ein Cycloalkylenrest mit 8 bis 18 C-Atomen, deren C-Ketten durch Ethersauerstoffatome unterbrochen sein können, oder ein Aralkylenrest mit 8 bis 16 C-Atomen, wobei die cycloaliphatischen und aromatischen Glieder durch eine Einfachbindung, durch - O -, - S -, - $SO_2$ -, - CO -

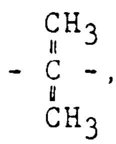 , 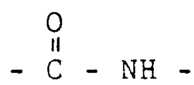 oder 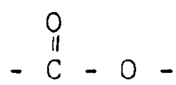

verbunden sein können, bedeuten.

**8.** Strahlungsempfindliche Verbindungen nach Anspruch 1, dadurch gekennzeichnet, daß die strahlungsempfindlichen Verbindungen Amide sind aus der 1,2-Naphthochinon-(2)-diazid-4-sulfonsäure der Formel I und einem primären oder sekundären Amin der Formel VI

$$H - NR_gR_h \qquad (VI)$$

in der

$R_g$ = Wasserstoff, ein ggf. substituierter Alkyl-, Cycloalkyl-, Aryl- oder Aralkylrest mit 1 bis 14 C-Atomen, dessen C-Kette durch Ethersauerstoffatome unterbrochen sein kann,

$R_h$ = - E - $NR_gH$

worin

E = ein Alkylenrest mit 2 bis 12 C-Atomen, dessen C-Kette durch Ethersauerstoffatome unterbrochen sein kann, ein Cycloalkylen-, Arylen- oder Aralkylenrest mit 8 bis 18 C-Atomen, wobei im Falle mehrkerniger Verbindungen die cycloaliphatischen und aromatischen Glieder durch eine Einfachbindung, durch - O -, - S -, - $SO_2$ -, - CO - oder - $R_g$ - verbunden sein können,

bedeuten.

**9.** Strahlungsempfindliches Gemisch, das ein in Wasser unlösliches, in wäßrig alkalischen oder organischen Lösemitteln lösliches, zumindest aber quellbares harzartiges Bindemittel, gegebenenfalls einen Vernetzer sowie eine strahlungsempfindliche Verbindung oder ein Gemisch strahlungsempfindlicher Verbindungen enthält, dadurch gekennzeichnet, daß mindestens eine der strahlungsempfindlichen Verbindungen ein Ester oder Amid nach einem der Ansprüche 1 bis 8 ist.

**10.** Gemisch nach Anspuch 9, dadurch gekennzeichnet, daß die strahlungsempfindliche Verbindung ein Ester nach Ansprüchen 1 bis 7 ist.

**11.** Gemisch nach Anspruch 9, dadurch gekennzeichnet, daß die strahlungsempfindliche Verbindung ein Amid nach Ansprüchen 1 bis 6 und 8 ist.

**12.** Gemisch nach Ansprüchen 9 bis 11, dadurch gekennzeichnet, daß das Bindemittel ein Novolak, ein Polyvinylphenol oder ein Polyvinylalkylphenol ist.

**13.** Gemisch nach Ansprüchen 9 bis 12, dadurch gekennzeichnet, daß das Gemisch ein Lösemittel bzw. ein Lösemittelgemisch enthält.

**14.** Gemisch nach Ansprüchen 9 bis 12, dadurch gekennzeichnet, daß das Gemisch zusätzlich Farbstoffe, Verlaufmittel, Weichmacher, Haftvermittler, Entwicklungsbeschleuniger und/oder Tenside enthält.

**15.** Gemisch nach Ansprüchen 9 bis 14, dadurch gekennzeichnet, daß das Bindemittel in einer Konzentration von 15 bis 99 Gewichtsprozent, bezogen auf die festen Bestandteile des Gemisches, vorhanden ist.

16. Gemisch nach Ansprüchen 9 bis 15, dadurch gekennzeichnet, daß die strahlungsempfindliche Verbindung in einer Konzentration von 1 bis 85 Gewichtsprozent, bezogen auf die festen Bestandteile des Gemisches, vorhanden ist.

17. Strahlungsempfindliches Aufzeichnungsmaterial, im wesentlichen bestehend aus einem Schichtträger und einer strahlungsempfindlichen Schicht, dadurch gekennzeichnet, daß die strahlungsempfindliche Schicht aus einem im wesentlichen von Lösemitteln befreiten strahlungsempfindlichen Gemisch nach Ansprüchen 9 bis 15 besteht.

18. Aufzeichnungsmaterial nach Anspruch 17, dadurch gekennzeichnet, daß der Schichtträger ein Silicium- oder Galliumarsenidwafer ist, auf dem zwischen dem halbleitenden Material und der strahlungsempfindlichen Schicht noch mindesens eine weitere Schicht aufgebracht ist.

## Claims

1. A novel radiation-sensitive compound which is an amide or ester of a 1,2-naphthoquinone-(2)-diazide-4-sulfonic acid of the general formula

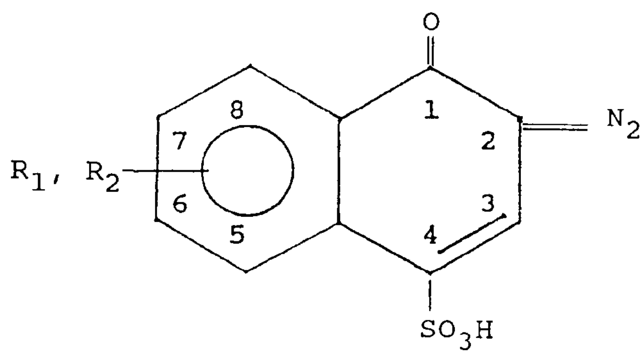

(I)

in which
$R_1$ und $R_2$ are identical or different and in the case of

**amides** denote hydrogen atoms, groups of alkyl, alkyl ether or alkyl thioether whose carbon chains may be interrupted by ether oxygen atoms, groups of acylamino or sulfonic acid ester,

**esters** additionally also denote sulfonamide groups

with the proviso that $R_1$ and $R_2$ are not being hydrogen at the same time.

2. A compound as claimed in claim 1, in which
$R_1$ and $R_2$ are identical or different and denote hydrogen, an alkyl group containing 1 to 6 carbon atoms, an alkyl ether group or alkyl thioether group whose carbon chains may be interrupted by ether oxygen atoms, containing a total of 1 to 6 carbon atoms, or an acylamino group containing 2 to 6 carbon atoms.

3. A compound as claimed in claim 1 or 2, in which
$R_1$ denotes hydrogen and
$R_2$ denotes alkyl ether groups or alkyl thioether groups whose carbon chain may be interrupted by ether oxygen atoms, containing 1 to 6 carbon atoms.

4. A compound as claimed in claim 1 or 2, in which
$R_1$ denotes hydrogen or methyl and
$R_2$ denotes methyl or an alkyl ether group containing 1-4 carbon atoms.

5. A compound as claimed in claims 1 to 4, in which
$R_1$ denotes, in position 6, hydrogen or methyl and
$R_2$ denotes, in position 7, methyl or the methylether group.

6. A compound as claimed in claims 1 to 4, in which

$R_1$ denotes, in position 6, hydrogen and
$R_2$ denotes, in position 7, the methylether group.

**7.** A radiation-sensitive compound as claimed in claim 1, which is an ester and is formed from the 1,2-naphthoquinone-(2)-diazide-4-sulfonic acid of the formula I and a mono- or polyhydroxyaryl compound of the formula II:

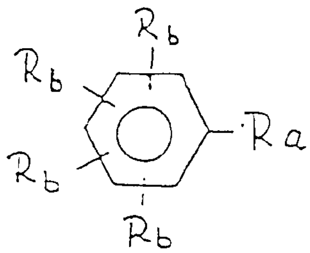

(II)

in which
$R_a$ = H, -X-$R_c$ or

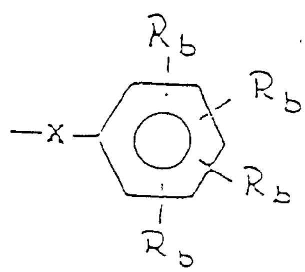

$R_b$ = H, an OH group, halogen or a lower alkyl group, at least one and not more than six $R_b$ groups being OH groups,
X = a carbon bond, -O-, -S-, -$SO_2$-,

$$-\underset{\underset{O}{\parallel}}{C}-,\ -\underset{\underset{O}{\parallel}}{C}-(CH_2)_n-,\ -CH_2-\underset{\underset{O}{\parallel}}{C}-(CH_2)_n,$$

$$-\underset{\underset{O}{\parallel}}{C}-(CH_2)_n-\underset{\underset{O}{\parallel}}{C}-,\ -\underset{\underset{O}{\parallel}}{C}-O-,\ -\underset{\underset{O}{\parallel}}{C}-O-(CH_2)_n-,$$

$$-CH_2-\underset{\underset{O}{\parallel}}{C}-O-(CH_2)_n-,\ -(CH_2)_p-,\ CH_3-\overset{|}{\underset{|}{C}}-CH_3,$$

$$CH_3-\overset{|}{\underset{|}{C}}-(CH_2)_n-Y,\ H-\overset{|}{\underset{|}{C}}-(CH_2)_n-Y$$

or

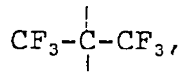

it being possible in each case for individual $CH_2$ groups to be replaced by ether functions or the hydrogen by substituents,

n = 1 or 2, P = 1 to 3,

Y = H or an optionally substituted alkoxy group, carboxy group, carboxyalkyl group, carboxyalkoxyalkyl group or aryl group, and

$R_c$ = H or an optionally substituted alkyl group or aryl group,

or

of the formula III

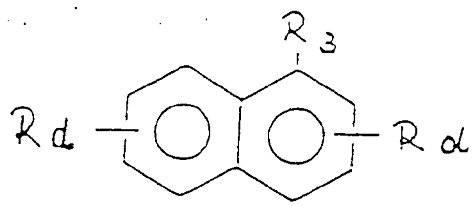

(III)

in which

$R_3$ = H or

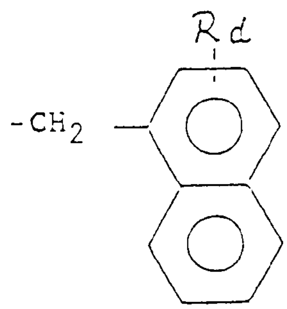

and

$R_d$ = H or an OH group, at least one of the $R_d$ groups being an OH group,

or of the formula IV

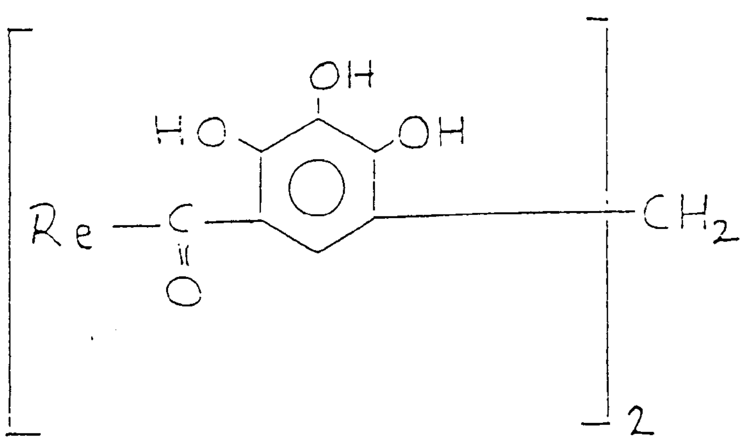

(IV)

in which

$R_e$ = H or an optionally substituted alkyl group, alkoxy group or aryl group,

or of the formula V

$R_f$-OH (V)

in which

$R_f$ = a straight-chain or branched alkyl group, cycloalkyl group or aralkyl group, optionally substituted by halogen or an acylamino radical and containing 1 to 14 carbon atoms, whose carbon chain may be interrupted by ether oxygen atoms,

or of the group

-Z-OH

in which

Z = an alkylene radical containing 2 to 12 carbon atoms or a cycloalkylene radical containing 8 to 18 carbon atoms, whose carbon chains may be interrupted by ether oxygen atoms, or an aralkylene radical containing 8 to 16 carbon atoms, it being possible for the cycloaliphatic and aromatic members to be joined by a single bond, by -O-, -S-, $-SO_2-$, -CO-

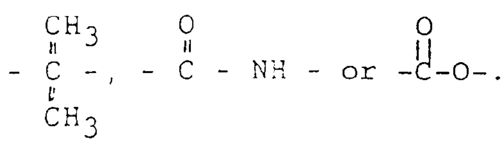

8. A radiation-sensitive compound as claimed in claim 1, which is an amide formed from the 1,2-naphthoquinone-(2)-diazide-4-sulfonic acid of the formula I and a primary or secondary amine of the formula VI:

$H-NR_gR_h$ (VI)

in which

$R_g$ = hydrogen, an optionally substituted alkyl, cycloalkyl, aryl or aralkyl radical containing 1 to 14 carbon atoms, whose carbon chain may be interrupted by ether oxygen atoms,

$R_h$ = $-E-NR_gH$

in which

E = an alkylene radical containing 2 to 12 carbon atoms, whose carbon chain may be interrupted by ether oxygen atoms, a cycloalkylene, arylene or aralkylene radical containing 8 to 18 carbon atoms, it being possible, in the case of polynuclear compounds, for the cycloaliphatic and aromatic members to be joined by a single bond, by -O-, -S-, $-SO_2-$, -CO- or $-R_g-$.

9. A radiation-sensitive mixture which contains a water-insoluble resinous binder which is soluble, or at least swellable, in aqueous alkaline or organic solvents, optionally a crosslinking agent and a radiation-sensitive compound or a mixture of radiation-sensitive compounds, wherein at least one of the radiation-sensitive compounds is an ester or amide as claimed in one of claims 1 to 8.

10. A mixture as claimed in claim 9, wherein the radiation-sensitive compound is an ester as claimed in claims 1 to 7.

11. A mixture as claimed in claim 9, wherein the radiation-sensitive compound is an amide as claimed in claims 1 to 6 and 8.

12. A mixture as claimed in claims 9 to 11, wherein the binder is a novolak, a polyvinylphenol or a polyvinylalkylphenol.

13. A mixture as claimed in claims 9 to 12, which contains a solvent or a solvent mixture.

14. A mixture as claimed in claims 9 to 12, which additionally contains dyestuffs, leveling agents, plasticizers, adhesion promoters, development accelerators and/or surfactants.

15. A mixture as claimed in claims 9 to 14, wherein the binder is present in a concentration of 15 to 99 percent by weight, based on the solid constituents of the mixture.

16. A mixture as claimed in claims 9 to 15, wherein the radiation-sensitive compound is present in a concentration of 1 to 85 percent by weight, based on the solid constituents of the mixture.

17. A radiation-sensitive copying material, essentially composed of a coating base and a radiation-sensitive coating, wherein the radiation-sensitive coating is composed of a radiation-sensitive mixture as claimed

in claims 9 to 15 essentially freed of solvent.

18. A copying material as claimed in claim 17, wherein the coating base is a silicon wafer or a gallium arsenide wafer on which at least one further coating is additionally deposited between the semiconducting material and the radiation-sensitive coating.

## Revendications

1. Composés sensibles aux radiations, qui représentent des amides ou des esters d'un acide 1,2-naphtoquinone-(2)-diazide-4-sulfonique de formule générale

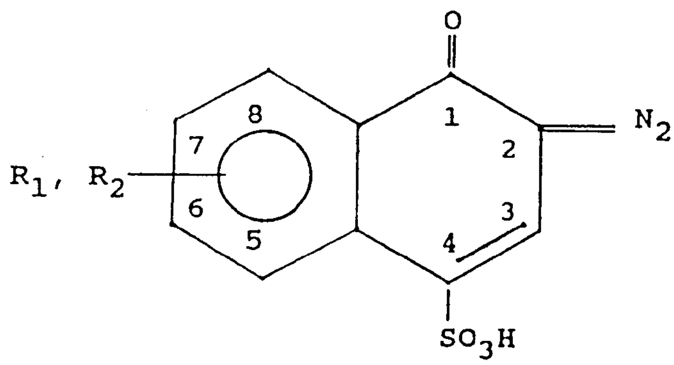

(I)

dans laquelle $R_1$ et $R_2$ sont identiques ou différents et, dans le cas

**d'amides** représentent des atomes d'hydrogène, des groupes alkyle, alkyléther ou thioalkyléther, dont les chaînes carbonées peuvent être interrompues par des atomes d'oxygène de fonction éther, des groupes acylamino, ester d'acide carboxylique ou ester d'acide sulfonique,

**d'esters** représentent en outre également des groupes sulfonamido,

étant entendu que $R_1$ et $R_2$ ne sont pas simultanément des atomes d'hydrogène.

2. Composés selon la revendication 1, dans lesquels
   $R_1$ et $R_2$ sont identiques ou différents et représentent un atome d'hydrogène ou un groupe alkyle ayant de 1 à 6 atomes de carbone, ou un groupe alkyléther ou alkylthioéther, dont les chaînes carbonées peuvent être interrompues par des atomes d'oxygène de fonction éther, et ayant au total de 1 à 6 atomes de carbone, ou un groupe acylamino ayant de 2 à 6 atomes de carbone.

3. Composés selon la revendication 1 ou 2, dans lesquels
   $R_1$ représente un atome d'hydrogène et
   $R_2$ représente des groupes alkyléther ou alkylthioéther, dont la chaîne carbonée peut être interrompue par des atomes d'oxygène de fonction éther, ayant de 1 à 6 atomes de carbone.

4. Composés selon la revendication 1 ou 2, dans lesquels
   $R_1$ représente un atome d'hydrogène ou le groupe méthyle et
   $R_2$ représente le groupe méthyle ou un groupe alkyléther ayant de 1 à 4 atomes de carbone.

5. Composés selon les revendications 1 à 4, dans lesquels
   $R_1$ en position 6 représente un atome d'hydrogène ou le groupe méthyle et
   $R_2$ en position 7 représente le groupe méthyle ou éther méthylique.

6. Composés selon les revendications 1 à 4, dans lesquels
   $R_1$ en position 6 représente un atome d'hydrogène et
   $R_2$ en position 7 représente le groupe éther méthylique.

7. Composés sensibles aux radiations selon la revendication 1, caractérisés en ce que les composés de type ester sensibles aux radiations sont formés à partir de l'acide 1,2-naphtoquinone-(2)-diazide-4-sulfonique

de formule I et d'un composé mono- ou polyhydroxyarylique de formule II

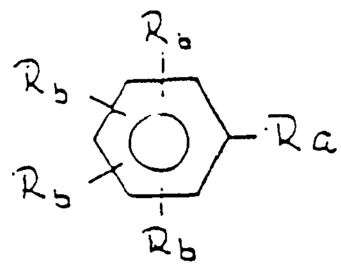

(II)

dans laquelle

$R_a$ = H, $-X-R_c$ ou

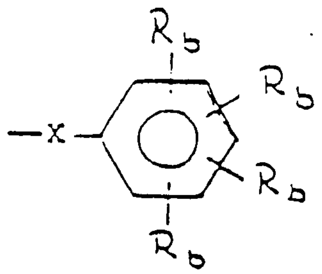

$R_b$ = H, le groupe OH, un atome d'halogène ou un groupe alkyle inférieur, au moins 1 et au maximum 6 groupes $R_b$ étant des groupes OH,

X représente une liaison carbone-carbone, -O-, -S-, $-SO_2-$,

$$-\underset{\underset{O}{\|}}{C}-,\quad -\underset{\underset{O}{\|}}{C}-(CH_2)_n-,\quad -CH_2-\underset{\underset{O}{\|}}{C}-(CH_2)_n,\quad -\underset{\underset{O}{\|}}{C}-(CH_2)_n-\underset{\underset{O}{\|}}{C}-,\quad -\underset{\underset{O}{\|}}{C}-O-,$$

$$-\underset{\underset{O}{\|}}{C}-O-(CH_2)_n-,\quad -CH_2-\underset{\underset{O}{\|}}{C}-O-(CH_2)_n-,\quad -(CH_2)_p-,\quad CH_3-\overset{|}{\underset{|}{C}}-CH_3,$$

$$CH_3-\overset{|}{\underset{|}{C}}-(CH_2)_n-Y,\quad H-\overset{|}{\underset{|}{C}}-(CH_2)_n-Y \text{ ou } CF_3-\overset{|}{\underset{|}{C}}-CF_3,$$

des groupes $CH_2$ individuels pouvant dans chaque cas être remplacés par des fonctions éther ou l'atome d'hydrogène pouvant dans chaque cas être remplacé par des substituants,

n = 1 ou 2,

p = 1 à 3,

Y = H ou un groupe alcoxy, carboxy, carboxyalkyle, carboxyalcoxyalkyle ou aryle, éventuellement substitué, et

$R_c$ = H ou un groupe alkyle ou aryle éventuellement substitué,

ou de formule III

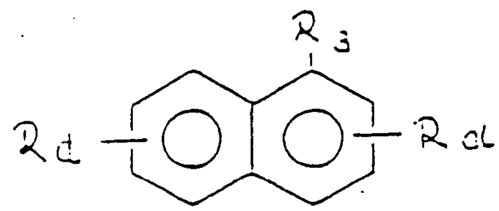

(III)

dans laquelle
$R_3$ = H ou

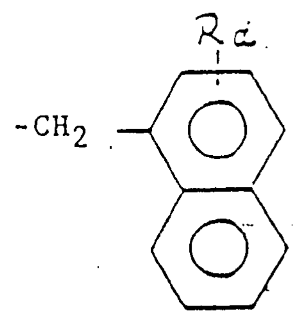

et
$R_d$ = H ou le groupe OH, au moins l'un des groupes $R_d$ étant le groupe OH,
ou de formule IV

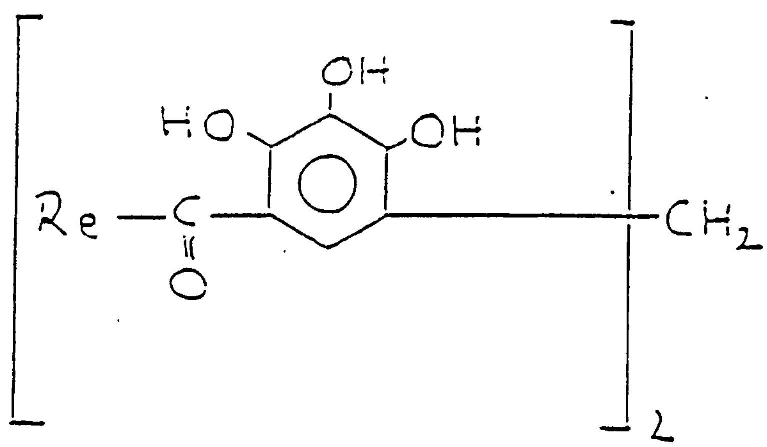

(IV)

dans laquelle
$R_e$ = H ou un groupe alkyle ou alcoxy éventuellement substitué, ou un groupe aryle,
ou de formule V

$R_f$-OH (V)

dans laquelle

$R_f$ représente un groupe alkyle, cycloalkyle ou aralkyle à chaîne droite ou ramifiée, ayant de 1 à 14 atomes de carbone et éventuellement substitué par un atome d'halogène ou par un radical acylamino, et dont la chaîne hydrocarbonée peut être interrompue par des atomes d'oxygène de fonction éther, ou correspondant au groupe -Z-OH dans lequel

Z représente un radical alkylène ayant de 2 à 12 atomes de carbone ou un radical cycloalkylène ayant de 8 à 18 atomes de carbone, dont les chaînes carbonées peuvent être interrompues par des atomes d'oxygène de fonction éther, ou un radical aralkylène ayant de 8 à 16 atomes de carbone, les chaînons cycloaliphatiques et aromatiques pouvant être reliés par une simple liaison, par -O-, -S-, $-SO_2-$, -CO-,

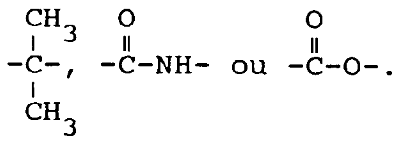

**8.** Composés sensibles aux radiations selon la revendication 1, caractérisés en ce que les composés sensibles aux radiations de type amide sont formés à partir de l'acide 1,2-naphtoquinone-(2)-diazide-4-sulfonique de formule I et d'une amine primaire ou secondaire de formule VI

$$H\text{-}NR_gR_h \qquad (VI)$$

dans laquelle

$R_g$ représente un atome d'hydrogène ou un radical alkyle, cycloalkyle, aryle ou aralkyle éventuellement substitué, ayant de 1 à 14 atomes de carbone, dont la chaîne carbonée peut être interrompue par des atomes d'oxygène de fonction éther,

$R_h$ = $-E\text{-}NR_gH$,

formule dans laquelle

E représente un radical alkylène ayant de 2 à 12 atomes de carbone, dont la chaîne carbonée peut être interrompue par des atomes d'oxygène de fonction éther, un radical cycloalkylène, arylène ou aralkylène ayant de 8 à 18 atomes de carbone, dans le cas de composés polycycliques, les chaînons cycloaliphatiques et aromatiques pouvant être reliés par une simple liaison, par -O-, -S-, $-SO_2-$, -CO- ou $-R_g-$.

**9.** Composition sensible aux radiations, qui contient un liant de type résine insoluble dans l'eau, soluble ou au moins susceptible de gonfler dans des solvants organiques ou aqueux-alcalins, éventuellement un agent de réticulation ainsi qu'un composé sensible aux radiations ou un mélange de composés sensibles aux radiations, caractérisée en ce qu'au moins l'un des composés sensibles aux radiations est un ester ou un amide selon l'une des revendications 1 à 8.

**10.** Composition selon la revendication 9, caractérisée en ce que le composé sensible aux radiations est un ester selon les revendications 1 à 7.

**11.** Composition selon la revendication 9, caractérisée en ce que le composé sensible aux radiations est un amide selon les revendications 1 à 6 et 8.

**12.** Composition selon les revendications 9 à 11, caractérisée en ce que le liant est une Novolaque, un polyvinylphénol ou un polyvinylalkylphénol.

**13.** Composition selon les revendications 9 à 12, caractérisée en ce que la composition contient un solvant ou un mélange de solvants.

**14.** Composition selon les revendications 9 à 12, caractérisée en ce que la composition contient en outre des colorants, agents d'étalement, plastifiants, tiers-solvants, accélérateurs de développement et/ou tensioactifs.

**15.** Composition selon les revendications 9 à 14, caractérisée en ce que le liant est présent à une concentration de 15 à 99 % en poids, par rapport aux composants solides de la composition.

**16.** Composition selon les revendications 9 à 15, caractérisée en ce que le composé sensible aux radiations est présent à une concentration de 1 à 85 % en poids, par rapport aux composants solide de la composition.

**17.** Matériau de reprographie sensible aux radiations, consistant essentiellement en un support de couche et une couche sensible aux radiations, caractérisé en ce que la couche sensible aux radiations est formé d'une composition sensible aux radiations, pratiquement privée de solvants, selon les revendications 9 à 15.

**18.** Matériau de reprographie selon la revendica tion 17, caractérisé en ce que le support de couche est une plaquette de silicium ou d'arsénure de gallium, sur laquelle est appliquée encore au moins une autre couche, entre le matériau semi-conducteur et la couche sensible aux radiations.

1,2-Naphthochinon-(2)-diazid-4-sulfonsäuren I

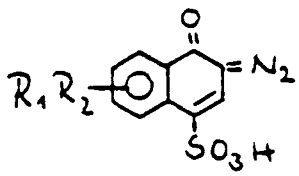

I

Tabelle 1

| Nr. der Verb. | $R_1$ | $R_2$ | N (%) ber. | N (%) gef. | S (%) ber. | S (%) gef. | Anmerkung |
|---|---|---|---|---|---|---|---|
| I/1 | 5-$OCH_3$ | H | 8,7 | 8,6 | 10,0 | 9,7 | Na-Salz |
| I/2 | 6-$OCH_3$ | H | 14,1 | 14,0 | 10,8 | 10,9 | $NH_4$-Salz |
| I/3 | 7-$OCH_3$ | H | 8,9 | 8,7 | 10,1 | 10,4 | |
| I/4 | 8-$OCH_3$ | H | 8,7 | 8,5 | 9,9 | 9,9 | Na-Salz |
| I/5 | 6-$CH_3$ | H | 15,0 | 14,7 | 11,4 | 11,2 | $NH_4$-Salz |
| I/6 | 6-$CH_3$ | 7-$CH_3$ | 14,2 | 14,1 | 10,9 | 10,8 | $NH_4$-Salz |
| I/7 | 7-$NHCOCH_3$ | H | 16,3 | 16,1 | 9,3 | 9,1 | $NH_4$-Salz + $H_2O$ |
| I/8 | 7-$SO_3H$ | H | 15,4 | 15,2 | 17,6 | 17,3 | $NH_4$-Salz |
| I/9 | 7-$SO_2N(CH_3)_2$ | H | 14,1 | 14,3 | 15,3 | 15,6 | $NH_4$-Salz + $H_2O$ |
| I/10 | 7-$SCH_3$ | H | 13,4 | 13,8 | 19,3 | 18,9 | $NH_4$-Salz + $H_2O$ |
| I/11 | 6-$COOCH_3$ | H | 12,2 | 12,2 | 9,3 | 9,9 | $NH_4$-Salz + $H_2O$ |
| I/12 | 6-Br | H | 11,5 | 11,3 | VERGLEICH | | $NH_4$-Salz + $H_2O$ |

Derivate der 1,2-Naphthochinon-(2)-diazid-4-sulfonsäure I

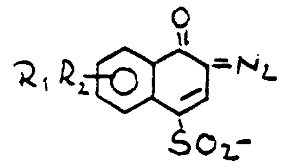 = D

Tabelle 2

| Nr. der Verb. | $R_1$ | $R_2$ | Derivat d. Diazosäure | N (%) ber. | N (%) gef. | S (%) ber. | S (%) gef. |
|---|---|---|---|---|---|---|---|
| 0 | H | H | 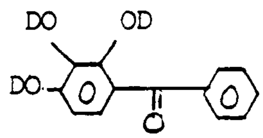 | VERGLEICH | | | |
| 1 | 7-$OCH_3$ | H | dto. | 8,3 | 8,0 | 9,4 | 9,4 |
| 2 | 8-$OCH_3$ | H | dto. | 8,3 | 7,9 | 9,4 | 9,1 |
| 3 | 6-$OCH_3$ | H | dto. | 8,3 | 7,9 | 9,4 | 9,0 |
| 4 | 6-$CH_3$ | H | dto. | 8,7 | 8,2 | 9,9 | 9,3 |
| 5 | 6-$CH_3$ | 7-$CH_3$ | dto. | 8,3 | 8,0 | 9,5 | 9,1 |
| 6 | 7-$OCH_3$ | H | 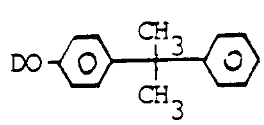 | 5,9 | 6,1 | 6,7 | 6,9 |
| 7 | 7-$OCH_3$ | H | 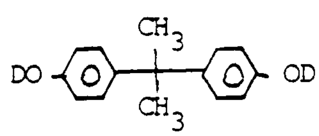 | 7,5 | 7,3 | 8,5 | 8,3 |

| Nr.d. Verb. | $R_1$ | $R_2$ | Derivat d. Diazosäure | N (%) ber. | N (%) gef. | S (%) ber. | S (%) gef. |
|---|---|---|---|---|---|---|---|
| 8 | 7-$OCH_3$ | H | 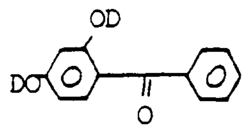 | 7,6 | 7,6 | 8,7 | 8,5 |
| 9 | 7-$OCH_3$ | H | 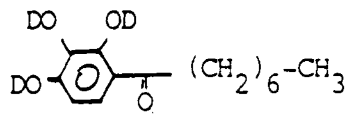 | 8,1 | 7,9 | 9,3 | 9,1 |
| 10 | 7-$OCH_3$ | H | 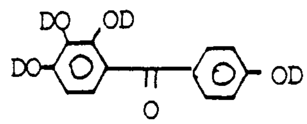 | 8,6 | 8,4 | 9,9 | 9,6 |
| 11 | 7-$OCH_3$ | H | 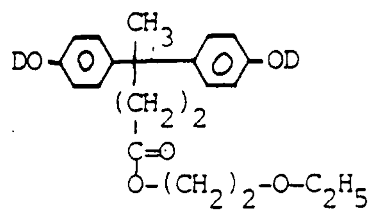 | 6,4 | 6,4 | 7,3 | 7,3 |
| 12 | 7-$OCH_3$ | H | 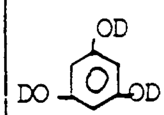 | 9,2 | 8,8 | 10,5 | 9,9 |
| 13 | 7-$OCH_3$ | H | 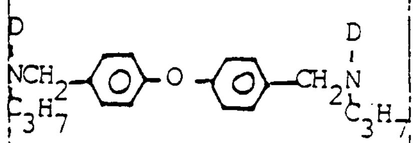 | 10,1 | 9,8 | 7,7 | 7,6 |
| 14 | 6-Br | H | 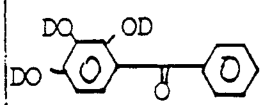 | | V E R | G L E | I C H |
| 15 | 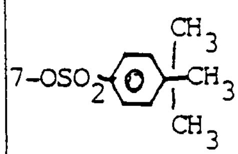 | H | 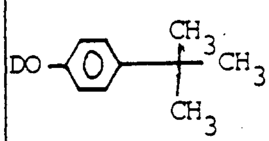 | 4,7 | 4,8 | 10,8 | 10,9 |
| 16 | 7-$NHCOCH_3$ | | 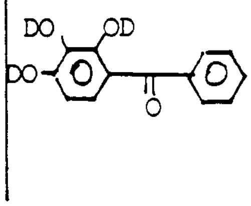 | 11,5 | 11,2 | 8,7 | 8,5 |

| Nr.d. Verb. | $R_1$ | $R_2$ | Derivat d. Diazosäure | N (%) | | S (%) | |
|---|---|---|---|---|---|---|---|
| | | | | ber. | gef. | ber. | gef. |
| 17 | 7-$SO_2N(CH_3)_2$ | H | 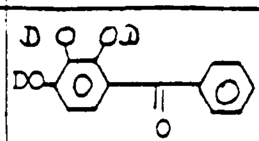 | 10,1 | 9,9 | 15,4 | 15,0 |
| 18 | 6-$COOCH_3$ | H | dto. | 7,6 | 7,2 | 8,7 | 8,0 |
| 19 | 6-$COOCH_3$ | H | 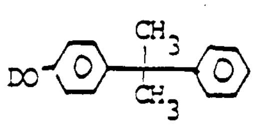 | 5,6 | 5,8 | 6,4 | 6,0 |